# EUROPEAN PATENT SPECIFICATION

(11) **EP 2 544 694 B1**
(45) Date of publication and mention of the grant of the patent: **31.10.2018**
(21) Application number: 11751270.7
(22) Date of filing: 02.03.2011
(51) Int. Cl.: A61K 31/7105, A61K 39/395, A61K 9/127, A61K 9/48, A61K 9/16, A61P 25/00, C07K 16/18

(54) **A METHOD FOR INHIBITING ALTERNATIVE PATHWAY MEDIATED DISORDERS WITH ANTI-PROPERDIN ANTIBODIES THAT INHIBIT C5 INTERACTIONS WITH PROPERDIN**
VERFAHREN ZUR HEMMUNG VON DURCH ALTERNATIVE PFADE VERMITTELTEN STÖRUNGEN MIT ANTIKÖRPERN GEGEN PROPERDIN ZUR HEMMUNG VON C5-INTERAKTIONEN MIT PROPERDIN
PROCÉDÉ POUR L'INHIBITION DE TROUBLES À MÉDIATION PAR LA VOIE ALTERNATIVE PAR DES ANTICORPS ANTI-PROPERDINE QUI INHIBENT LES INTERACTIONS DE C5 AVEC LA PROPERDINE

(30) Priority: 02.03.2010 US 309705 P
(43) Date of publication of application: 16.01.2013
(73) Proprietor: Novelmed Therapeutics, Inc., Cleveland, OH 44106 (US)
(72) Inventor: BANSAL, Rekha, Twinsburg OH 44087 (US)
(74) Representative: Maiwald Patent- und Rechtsanwaltsgesellschaft mbH
(86) International application number: PCT/US2011/026841
(87) International publication number: WO 2011/109494

(56) References cited:
- WO-A1-99/10009
- WO-A1-2006/128006
- WO-A1-2009/110918
- US-A1- 2004 137 514
- US-A1- 2008 233 113
- US-A1- 2009 017 031
- US-B2- 7 365 167
- WARK K L ET AL: "Latest technologies for the enhancement of antibody affinity", ADVANCED DRUG DELIVERY REVIEWS, ELSEVIER, AMSTERDAM, NL, vol. 58, no. 5-6, 7 August 2006 (2006-08-07), pages 657-670, XP024892147, ISSN: 0169-409X, DOI: 10.1016/J.ADDR.2006.01.025 [retrieved on 2006-08-07]

### SEQUENCE LISTING

<110> NovelMed Therapeutics
<120> A METHOD FOR INHIBITING ALTERNATIVE PATHWAY MEDIATED DISORDERS WITH ANTI-PROPERDIN ANTIBODIES THAT INHIBIT C5 INTERACTIONS WITH PROPERDIN
<130> NMT-019646WO ORD
<160> 15
<170> PatentIn version 3.5
<210> 1
   <211> 12
   <212> PRT
   <213> Mus musculus x Rattus norvegicus
<400> 1
   Gly Phe Ser Leu Ser Thr Ser Gly Met Gly Val Gly
   1               5                   10
<210> 2
   <211> 16
   <212> PRT
   <213> Mus musculus x Rattus norvegicus
<400> 2
   His Ile Trp Trp Asp Asp Val Lys Ser Tyr Asn Pro Ala Leu Lys Ser
   1 5 10 15
<210> 3
   <211> 10
   <212> PRT
   <213> Mus musculus x Rattus norvegicus
<400> 3
   Ile Gly Asp Gly Tyr Tyr Ser Phe Asp Tyr
   1 5 10
<210> 4
   <211> 11
   <212> PRT
   <213> Mus musculus x Rattus norvegicus
<400> 4
   Lys Ala Ser Gln Asp Val Ser Asp Ala Val Ala
   1 5 10
<210> 5
   <211> 7
   <212> PRT
   <213> Mus musculus x Rattus norvegicus
<400> 5
   Ser Pro Ser Tyr Arg Tyr Thr
   1 5
<210> 6
   <211> 9
   <212> PRT
   <213> Mus musculus x Rattus norvegicus
<400> 6
   Gln Gln His Tyr Ser Thr Pro Trp Thr
   1 5
<210> 7
   <211> 120
   <212> PRT
   <213> Homo sapiens
<400> 7
   Glu Val Gln Leu Val Gln Ser Gly Ala Glu Val Lys Lys Pro Gly Glu
   1 5 10 15
   Ser Leu Arg Ile Ser Cys Lys Gly Ser Gly Phe Ser Leu Ser Thr Ser
   20 25 30
   Gly Met Gly Val Gly Trp Ile Arg Gln Ser Pro Ser Arg Gly Leu Glu
   35 40 45
   Trp Leu Gly His Ile Trp Trp Asp Asp Val Lys Ser Tyr Asn Pro Ala
   50 55 60
   Leu Lys Ser Arg Val Thr Ile Ser Ala Asp Lys Ser Ile Ser Thr Ala
   65 70 75 80
   Tyr Leu Gln Trp Ser Ser Leu Lys Ala Ser Asp Thr Ala Met Tyr Tyr
   85 90 95
   Cys Ala Arg Ile Gly Asp Gly Tyr Tyr Ser Phe Asp Tyr Trp Gly Gln
   100 105 110
   Gly Thr Leu Val Thr Val Ser Ser
   115 120
<210> 8
   <211> 111
   <212> PRT
   <213> Homo sapiens
<400> 8
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asp Val Ser Asp Ala
               20                  25                  30
   Val Ala Trp Tyr Gln Gln Lys Pro Gly Lys Ala Pro Lys Leu Leu Ile
           35                  40                  45
   Tyr Ser Pro Ser Tyr Arg Tyr Thr Gly Val Pro Ser Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln His Tyr Ser Thr Pro Trp
                   85                  90                  95
   Thr Phe Gly Gln Gly Thr Lys Val Glu Ile Lys Arg Thr Val Ala
               100                 105                 110
<210> 9
   <211> 120
   <212> PRT
   <213> Homo sapiens
<400> 9
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Phe Ser Leu Ser Thr Ser
               20                  25                  30
   Gly Met Gly Val Gly Trp Val Arg Gln Ala Thr Gly Gln Gly Leu Glu
           35                  40                  45
   Trp Met Gly His Ile Trp Trp Asp Asp Val Lys Ser Tyr Asn Pro Ala
       50                  55                  60
   Leu Lys Ser Arg Val Thr Ile Thr Ala Asp Lys Ser Thr Ser Thr Ala
   65                  70                  75                  80
   Tyr Met Glu Leu Ser Ser Leu Arg Ser Glu Asp Thr Ala Val Tyr Tyr
                   85                  90                  95
   Cys Ala Arg Ile Gly Asp Gly Tyr Tyr Ser Phe Asp Tyr Trp Gly Gln
               100                 105                 110
   Gly Thr Leu Val Thr Val Ser Ser
           115                 120
<210> 10
   <211> 111
   <212> PRT
   <213> Homo sapiens
<400> 10
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Val Ser Asp Ala
               20                  25                  30
   Val Ala Trp Tyr Gln Gln Lys Pro Gly Lys Ala Pro Lys Leu Leu Ile
           35                  40                  45
   Tyr Ser Pro Ser Tyr Arg Tyr Thr Gly Ile Pro Pro Arg Phe Ser Gly
       50                  55                  60
   Ser Gly Tyr Gly Thr Glu Phe Thr Phe Thr Ile Ser Ser Leu Glu Ala
   65                  70                  75                  80
   Glu Asp Ala Ala Thr Tyr Tyr Cys Gln Gln His Tyr Ser Thr Pro Trp
                   85                  90                  95
   Thr Phe Gly Gln Gly Thr Lys Val Glu Ile Lys Arg Thr Val Ala
               100                 105                 110
<210> 11
   <211> 120
   <212> PRT
   <213> Homo sapiens
<400> 11
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Ser Leu Ser Thr Ser
               20                  25                  30
   Gly Met Gly Val Gly Trp Val Arg Gln Ala Pro Gly Lys Gly Leu Glu
           35                  40                  45
   Trp Val Ser His Ile Trp Trp Asp Asp Val Lys Ser Tyr Asn Pro Ala
       50                  55                  60
   Leu Lys Ser Arg Phe Thr Ile Ser Arg Asp Asn Ala Lys Asn Ser Leu
   65                  70                  75                  80
   Tyr Leu Gln Met Asn Ser Leu Arg Ala Glu Asp Thr Ala Val Tyr Tyr
                   85                  90                  95
   Cys Ala Lys Ile Gly Asp Gly Tyr Tyr Ser Phe Asp Tyr Trp Gly Gln
                   100                 105                 110
   Gly Thr Leu Val Thr Val Ser Ser
           115                 120
<210> 12
   <211> 111
   <212> PRT
   <213> Homo sapiens
<400> 12
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Val Ser Asp Ala
               20                  25                  30
   Val Ala Trp Tyr Gln Gln Lys Pro Gly Lys Ala Pro Lys Leu Leu Ile
           35                  40                  45
   Tyr Ser Pro Ser Tyr Arg Tyr Thr Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Gln His Tyr Ser Thr Pro Trp
                   85                  90                  95
   Thr Phe Gly Gln Gly Thr Lys Val Glu Ile Lys Arg Thr Val Ala
               100                 105                 110
<210> 13
   <211> 120
   <212> PRT
   <213> Homo sapiens
<400> 13
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Thr Ser
               20                  25                  30
   Gly Met Gly Val Gly Trp Val Arg Gln Ala Pro Gly Lys Gly Leu Glu
           35                  40                  45
   Trp Val Gly His Ile Trp Trp Asp Asp Val Lys Ser Tyr Asn Pro Ala
       50                  55                  60
   Leu Lys Ser Arg Phe Thr Phe Ser Leu Asp Thr Ser Lys Ser Thr Ala
   65                  70                  75                  80
   Tyr Leu Gln Met Asn Ser Leu Arg Ala Glu Asp Thr Ala Val Tyr Tyr
                   85                  90                  95
   Cys Ala Lys Ile Gly Asp Gly Tyr Tyr Ser Phe Asp Tyr Trp Gly Gln
               100                 105                 110
   Gly Thr Leu Val Thr Val Ser Ser
           115                 120
<210> 14
   <211> 111
   <212> PRT
   <213> Homo sapiens
<400> 14
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Val Ser Asp Ala
               20                  25                  30
   Val Ala Trp Tyr Gln Gln Lys Pro Gly Lys Ala Pro Lys Val Leu Ile
           35                  40                  45
   Tyr Ser Pro Ser Tyr Arg Tyr Thr Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Ser Thr Pro Trp
                   85                  90                  95
   Thr Phe Gly Gln Gly Thr Lys Val Glu Ile Lys Arg Thr Val Ala
               100                 105                 110
<210> 15
   <211> 24
   <212> PRT
   <213> Homo sapiens
<400> 15
   Ala Phe Ala Tyr Gln Lys Arg Ser Gly Gly Leu Cys Gln Pro Cys Arg
   1               5                   10                  15
   Ser Pro Arg Trp Ser Leu Trp Ser
               20

## Description

### FIELD OF THE INVENTION

The present invention relates to the use of anti-properdin antibody agents that inhibit properdin binding to a complement protein, for example, C5 or C5b, to selectively inhibit alternative complement pathway function in mammals in prophylactic and therapeutic settings to prevent, halt or reverse the disease process.

### BACKGROUND OF THE INVENTION

The complement system is important for host defense against pathogens and is involved in clearance of, for example, pathogens, bacterial cells, foreign antigens, and tumor cells. The alternative complement pathway (AP) is activated as a result of both pathological inflammation and autoimmune diseases. Elevated levels of C3a, C5a, and C5b-9 have been found to be associated with several acute and chronic diseases. Therefore, inhibition of disease-induced AP activation is important for clinical benefit in the diseases where complement activation plays a role in disease pathology.

The complement system is activated via three distinct complement pathways; the classical, the lectin and the alternative pathways. The classical pathway is activated via antigen-antibody complexes and is required for host defense. The lectin pathway is a variation of the classical pathway. The alternative pathway is activated by foreign material, artificial surfaces, dead tissues, bacteria, and dead yeast cells. Therapeutic strategies that selectively inhibit activation of the alternative pathway without affecting the classical pathway are of disease relevance to treating diseases and disorders acsccoiated with AP activation. Under pathological conditions, activation of the alternative pathway generates anaphylatoxins, including C3a, C5a and the tissue damaging C5b-9 molecule also known as MAC. These molecules mediate inflammation and tissue damage via cellular activation, release of inflammatory mediators, and deposition of C5b-9. Cellular leukocyte chemotaxis and activation of macrophages, neutrophils, platelets, mast cells and endothelial cells provide evidence for the inflammatory response. Additionally, vascular permeability, cytolysis, and tissue injury processes also occur during inflammation. Activated cells release inflammatory mediators such as TNF-α, IL-1β, IL-6, IL-8, VEGF, neutrophil elastase, and peroxide.

It is known that properdin is required for the initiation of the alternative complement pathway, since its binding to C3b constitutes the necessary first step in AP activation. Properdin binds C3b with high affinity. Properdin-bound C3b subsequently binds with factor B to form the PC3bB complex, which is then cleaved by factor D to form the PC3bBb complex, where Ba is released. As additional molecules of C3b and Bb are produced, their assembly into the PC3bBb complex forms C5 convertase, which cleaves C5 into C5a and C5b. The so-formed C5 represents an anchor protein that forms the C5b-9 complex upon association with C6, C7, C8, and C9. Properdin concentration in blood is nearly 5 µg/mL, and, thus, it is the only non-protease molecule present at a much lower concentration than Factor D.

Properdin is an alternative pathway specific molecule, whose presence and function are required for the propagation of the alternative pathway. Thus, inhibition of AP activation without inhibiting the classical complement pathway appears to be an important therapeutic strategy to inhibit the disease-induced activation of the AP. Hence, blocking, inhibiting or preventing AP activation via depleting properdin, neutralizing properdin or inactivating properdin remains potentially important therapeutic strategies.

No anti-properdin antibodies that bind to C5 or C5b have previously been discovered. The antibodies of the present invention can inhibit properdin binding to C5 or C5b, and thus prevent the formation of C3a, C3b, C5a, C5b, and C5b-9. Similarly, anti-C5 antibodies can be generated that would inhibit properdin interaction to C5/C5b and prevent the formation of C3a, C3b, C5a, C5b, and C5b-9.

Since the binding affinity of properdin to C5 is weaker than the binding affinity of the antibodies of the present invention to properdin, the antibodies of the present invention can inhibit the binding of C5 molecules to properdin and can provide a therapeutic benefit by preventing AP activation in a setting where the binding of properdin to C5 is preexisting. Treatment with anti-properdin antibodies that specifically prevent the binding of properdin to C5 can provide significant therapeutic benefit in pathological conditions where alternative complement pathway is activated.

WO 2009/110918 A1 discloses antibodies against properdin.

### SUMMARY OF THE INVENTION

Antibodies that can inhibit the binding of properdin to C3b have been developed, and those that de-polymerize polymeric properdin have been discussed. These antibodies can inhibit the formation of C3a, C5a, and C5b-9.

The present invention relates to the use of an antibody or fragment thereof for inhibiting alternative complement pathway activation in the blood of a mammal, the antibody or fragment thereof specifically binding to properdin and inhibiting the alternative complement pathway activation in the mammal by inhibiting the properdin binding to C5, wherein the antibody or fragment thereof comprises a heavy chain variable domain comprising the three CDRs of SEQ ID NO: 1, SEQ ID NO: 2 and SEQ ID NO: 3 and a light chain variable domain comprising the three CDRs of SEQ ID NO: 4, SEQ ID NO: 5 and SEQ ID NO: 6, wherein said antibody binds to properdin with a binding affinity of < 100pM

The present invention also relates to the use of antibodies that inhibit properdin binding to, for example, C5 or C5b and, as a result, the formation of C3a, C3b, C5a, C5b, and C5b-9.

In one aspect of the disclosure, the anti-properdin agent can bind to at least one complement protein, including, but not limited to, C5/C5b, C6, C7, C8, or C9.

In another aspect of the disclosure, the anti-properdin agent can be selected from the following group including, but not limited to: synthetic small molecules, aptamers of DNA, DNA fragments, SiRNA, monoclonal and polyclonal antibodies, and derivatives of the monoclonal and or polyclonal antibodies that block the binding of properdin to at least one of C5/C5b, C6, C7, C8, or C9, or any portion of the peptide similar to that found in at least one of C5/C5b, C6, C7, C8, or C9.

In a further aspect of the invention, the anti-properdin agent can be an anti-properdin antibody that is registered as PTA-9641 (14G11).

The anti properdin monoclonal antibody used in the the present invention binds the N terminal domain of SEQ ID NO: 15 AFAYQKRSGGLCQPCRSPRWSLWS. Such antibodies inhibit properdin binding to C5b and prevent AP activation.

In another aspect of the invention, the anti-properdin agent can be administered *in vivo* or *ex vivo* to a mammal, including a human.

In yet another aspect of the invention, the anti-properdin antibody can be a monoclonal, polyclonal, chimeric, recombinant, humanized, de-immunized or Humaneered™ antibody.

In one aspect of the invention, the anti-properdin antibody can also be an antibody fragment, such as a single chain antibody, IgG, F(ab')2, F(ab), F(ab') fragment, or truncated antibody, for example, an Fc truncated antibody.

In another aspect of the invention, the ratio of binding of the anti-properdin agent to properdin can be about 0.2:1 to about 1.5:1.

In a further aspect of the invention, the prevention of properdin binding to at least one complement protein can allow for the inhibition of the alternative complement pathway in the mammal without affecting classical pathway activation.

In yet another aspect of the invention, the anti-properdin antibody reduces the level of free properdin in a mammal.

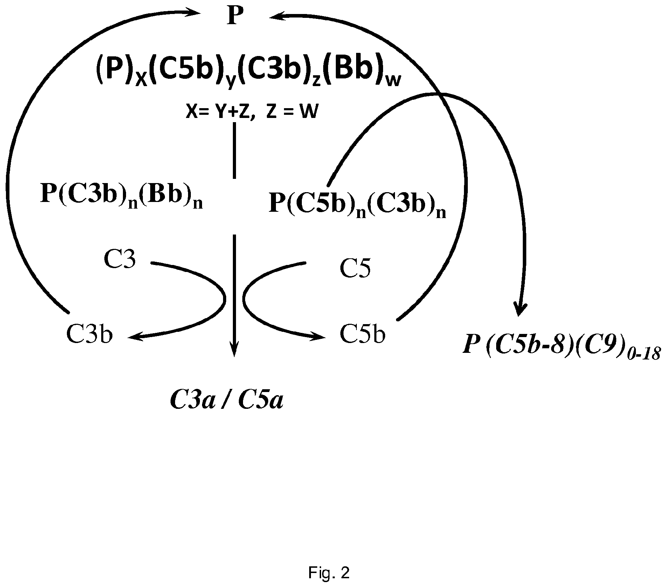

In one aspect of the invention, the anti-properdin antibody specifically binds properdin to prevent the binding of properdin to C5. The antibody can bind to an epitope on the properdin monomer at its C5 binding site and prevents the formation of oligomers of Properdin and C3b shown in Figure 2.

In another aspect of the invention the anti-properdin antibody specifically binds properdin to prevent the binding properdin to C6, C7, C8, or C9.

In a further aspect of the invention, an anti-properdin antibody generated in a mouse can be prepared with a characteristic CDR(s) region that binds and inhibits properdin binding to C5 and C9.

In yet another aspect of the invention, the anti-properdin antibody can inhibit the formation of the C5b-9 complex, also known as MAC.

In one aspect of the disclosure, the disclosure provides a method to ameliorate a disease or condition associated with excessive or uncontrolled alternative pathway complement activation by administering a therapeutically effective amount of an anti-properdin agent that specifically binds to properdin to inhibit the binding of properdin to C5.

In another aspect of the invention, the antibody can be produced in a cell or mammal or it can be genetically engineered in a mammal, phage display or from a recombinant or natural antibody library.

In a further aspect of the invention, administration of the anti-properdin antibody or an antigen binding fragment to a subject can result in at least one of the following: inhibition of properdin binding to C5, inhibition of properdin binding to C5b, inhibition of properdin binding to C9, reduction in the formation of C3bBb, reduction in the production of C3a and C5a, reduction in the formation of C5b-9 complex, reduction in the activation of neutrophils, reduction in the activation of monocytes reduction in the activation of platelets, reduction in the formation of leukocyte-platelet conjugates, reduction in TNF-alpha production, and the reduction in production of neutrophil elastase. Consequently, the antibody or fragment thereof can also inhibit the alternative pathway dependent rabbit erythrocyte lysis in human serum/plasma.

In one aspect of the disclosure, the disclosure relates to a method of inhibiting alternative complement pathway activation in a mammalian host that includes the administration of a therapeutically effective amount of an anti-properdin monoclonal antibody that specifically binds to an epitope on properdin, where the epitope is involved in C5, C5b or C9 binding.

In another aspect, the disclosure relates to a method of inhibiting alternative complement pathway activation without inhibiting the classical pathway activation in a mammal by administering in a mammal a therapeutically effective amount of an antibody that specifically binds to an epitope of properdin, which blocks the alternative pathway activation via the inhibition of properdin and C5 interaction without affecting the classical pathway activation.

In a further aspect, the present disclosure relates to a method of inhibiting alternative complement pathway activation without inhibiting the classical pathway activation in a mammal. The method includes the step of administering to a mammal a therapeutically effective amount of an antibody that specifically binds to properdin and inhibits deposition of C5b-9, which blocks the alternative pathway activation via the inhibition of properdin and C5 interaction without affecting the classical pathway activation.

In another aspect, the present disclosure relates to a method of inhibiting alternative complement pathway activation, including the step of administering to a mammal a therapeutically effective amount of an antibody blocks the formation of the PC3b complex, the PC3bB complex, and the PC3bBb complex via the inhibition of properdin and C5 interaction without affecting the classical pathway activation.

In yet another aspect, the present disclosure can provide a method of inhibiting activation of the alternative complement pathway in an individual who is at risk of developing a disease or a condition in which activation of the alternative complement pathway contributes to the disease pathology, exacerbates the disease, or causes the disease, via the administration of any specific antibodies of the present invention or antigen-binding fragments thereof disclosed herein to an individual in need.

In a further aspect, the present disclosure can relate to a method of treating alternative pathway activation mediated by disease-related or pathological conditions including the step of administering to a mammal a therapeutically effective amount of an antibody or fragment thereof that specifically binds to properdin and blocks the initiation of C9 assembly and prevents the formation of C5b-9 complex formation at the site of pathology.

In another aspect of the disclosure, the present disclosure provides an anti-properdin agent in a pharmaceutically acceptable carrier which is selected from the group comprising: a dry, dispersible powder; anhydrous ethanol; small capsules; liposomes; a nebulized spray; and an injectable excipient.

In a further aspect of the invention, the anti-properdin antibody can specifically bind to an epitope on the properdin monomer at its C5 binding site and can prevent the formation of polymers of Properdin and C3b.

In one aspect of the disclosure, the present disclosure relates to a method of making a rat or mouse anti-properdin monoclonal antibodies in a properdin-knockout mice or other rodent. The method includes the step of administering to a mouse or other rodent an effective amount of properdin or fragment thereof to the properdin knockout an effective amount of the properdin or fragment thereof that causes the knockout rodent to produce antibodies against properdin. The mouse or other rodent can include human immunoglobulin genes. The fragment includes a peptide having a C5 binding domain.

In another aspect of the disclosure, the present disclosure also relates to an isolated antibody or antigen binding portion thereof that binds to human properdin and blocks C5 or C5b binding to properdin. The antibody or antigen binding portion thereof comprises of a heavy chain variable domain that includes the amino acid sequences of the three CDRs (SEQID NO: 1) and a light chain variable domain that includes the amino acid sequences of the three CDRs (SEQ ID NO: 2).

In a further aspect of the invention, the anti-properdin antibody or antigen binding fragment thereof can comprise the heavy chain variable regions and the light chain variable regions of an antibody produced by the hybridoma cell line deposited under ATCC Accession Number PTA-9641.

In one aspect of the disclosure, the present disclosure further relates to an isolated antibody or antigen binding portion thereof that binds to human properdin. The antibody or antigen binding fragment thereof comprises of a heavy chain variable domain containing the amino acid sequences of the three CDRs in SEQ ID NO: 1.

In another aspect of the disclosure, the present disclosure further relates to an isolated antibody or antigen binding portion thereof that binds to human properdin. The antibody or antigen binding fragment thereof comprises of a light chain variable domain comprising the amino acid sequences of the three CDRs in SEQ ID NO: 2.

In yet another aspect of the disclosure, the anti-properdin antibody or antigen binding fragment thereof can also inhibit alternative pathway dependent rabbit erythrocyte lysis in human serum/plasma.

In one aspect of the disclosure, the present disclosure relates to a method of inhibiting alternative pathway complement activation in a mammal by the administration of a therapeutically effective amount of an anti-properdin antibody or antigen binding portion thereof to the mammal that specifically binds to properdin to inhibit alternative pathway complement activation, at a molar ratio of antibody to properdin monomer of about 0.2:1.

In another aspect of the invention, the antibody used in the present invention can be administered *in vivo* or *ex vivo.*

In a further aspect of the invention, the antibody or antigen binding portion thereof can specifically bind to an epitope on properdin and, consequently, inhibit the alternative complement pathway in the mammal.

In another aspect, the present disclosure can provide a method of treating a disease or disorder in which the AP activation plays a role. The treatment comprises of the step of administering an anti properdin antibody or antigen-binding fragment thereof derived from murine monoclonal antibody (ATCC PTA-9641) to a mammal that selectively binds to properdin at the binding site of C5or C5b and inhibits formation of the C3bBb complex, the PC3bBb complex, the PC5 complex, the PC9 complex, and the PC3b complex. The anti-properdin antibody or antigen-binding fragment thereof can have an equilibrium dissociation constant between about 1 nM to about 1 pM. The antibodies of the present disclosure can be administered to a mammal that has, or is at risk of developing a disease or disorder in which AP activation plays a role. In certain embodiments, the disease or disorder is selected from the group of multiple diseases, described herein, where alternative pathway plays a pathological role in the disease.

### BRIEF DESCRIPTION OF THE DRAWINGS

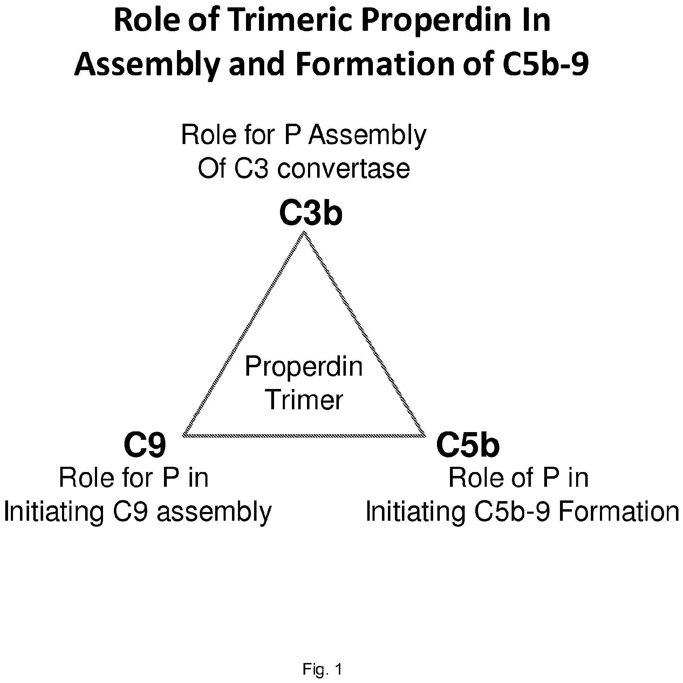

FIG. 1 illustrates the schematics of the hypothetical role of properdin in the assembly and formation of C5b-9.
FIG. 2 shows the formation of PC5bC3bBb convertase where if properdin can exist as a trimer.
FIG. 3 shows the binding affinity of properdin to C3b, C5/C5b, and C9
FIG. 4 shows the anti-properdin agent of the present invention can inhibit the hemolysis of rRBC.
FIG. 5 shows that both anti properdin IgG and F(ab')₂ bind properdin with high affinity.
FIG. 6 shows the inhibition of properdin binding to C5b and C9 by the anti-properdin antibody.
FIG. 7 shows the inhibition of AP-dependent formation of newly formed C3b in normal human serum.
FIG. 8 shows the inhibition of AP-dependent formation of PC3b complex in normal human serum.
FIG. 9 shows the inhibition of AP-dependent Bb formation in normal human serum.
FIG. 10 shows the inhibition of AP-dependent C5 formation in normal human serum.
FIG. 11 shows the inhibition of AP-dependent C9 formation in normal human serum.
FIG. 12 shows inhibition of AP-dependent C5b-9 formation in normal human serum.

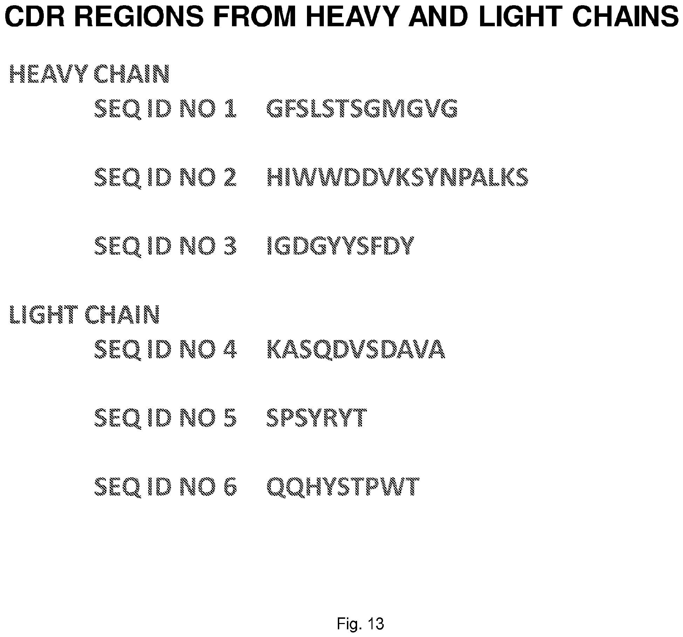

FIG. 13 shows SEQ ID NOs: 1-6.

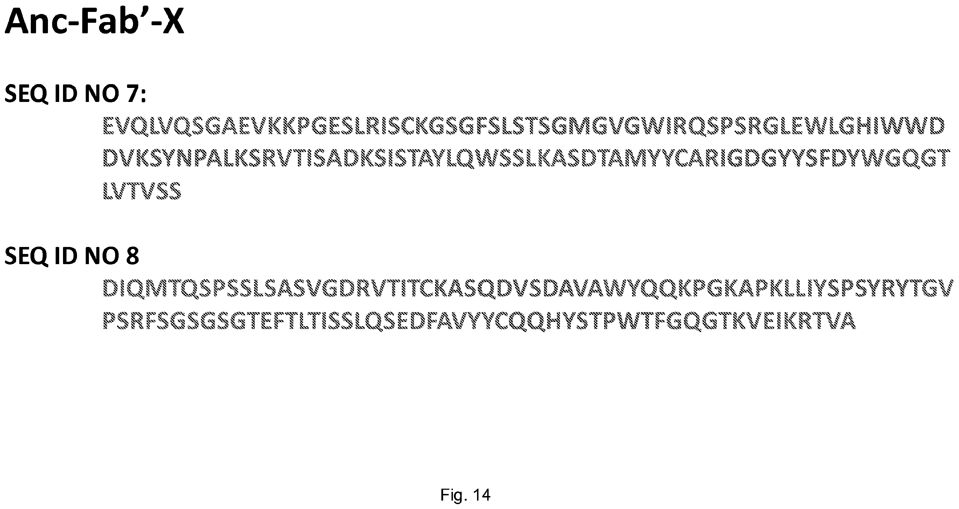

FIG. 14 shows SEQ ID NOs: 7-8.

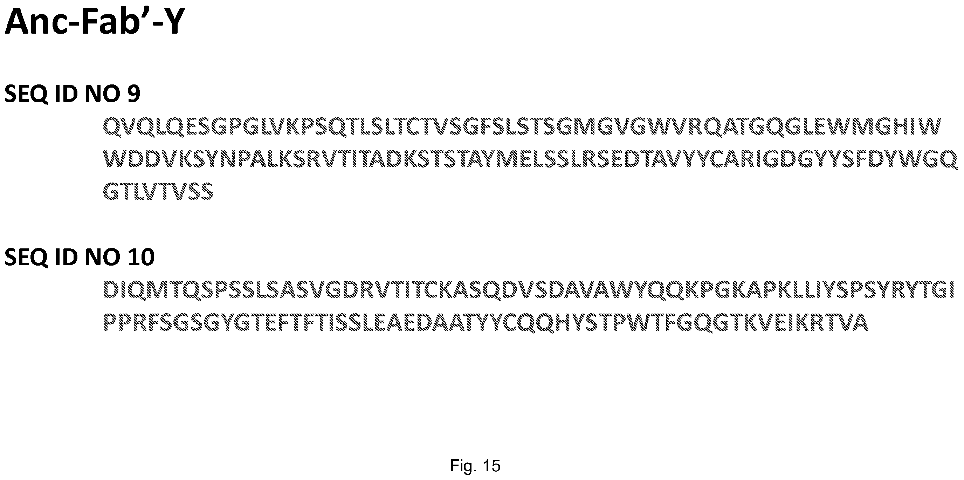

FIG. 15 shows SEQ ID NOs: 9-10.

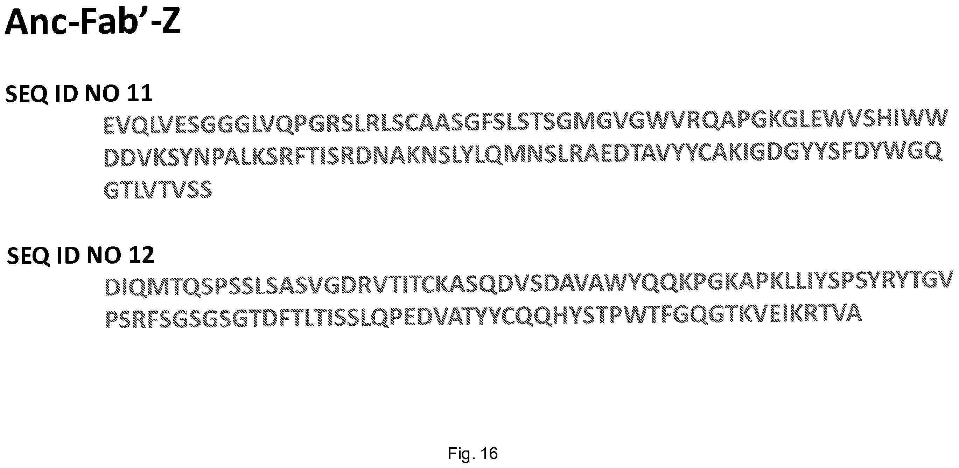

FIG. 16 shows SEQ ID NOs: 11-12.

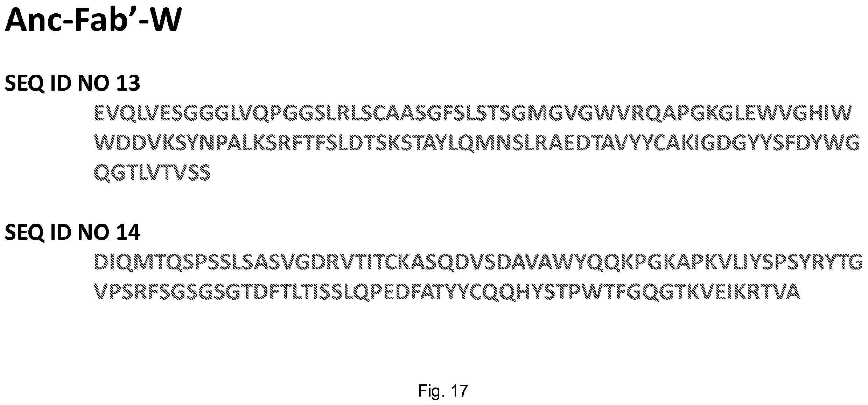

FIG. 17 shows SEQ ID NOs: 13-14.

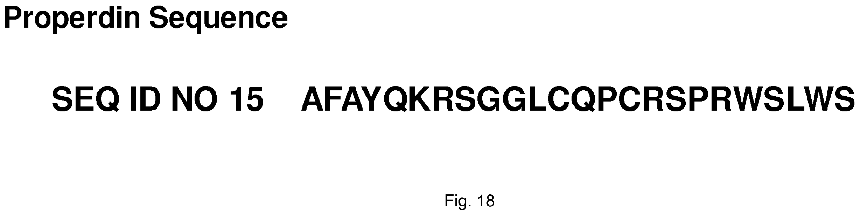

FIG. 18 shows SEQ ID NO: 15.

### DETAILED DESCRIPTION

As used here, the term "antibody" or "antibody fragments thereof' refer to an intact antibody, or a binding fragment thereof that competes with the intact antibody for specific binding sites. The binding fragments include Fab, Fab', F(ab')₂, Fv, and single-chain antibodies.

As used herein, the term "anti-properdin agent" refers to small molecules, DNA fragments, SiRNA, aptamers of DNA, monoclonal and polyclonal antibodies, and derivatives of the monoclonal and or polyclonal antibodies that block the binding of properdin to C5 or any portion of the peptide similar to that found in C5.

As used herein, the term "complementary determining region (CDR)" refers to regions on the antibody where a specific antibody can bind to.

As used herein, the term "conservative sequence modifications" refers to amino acid modifications that do not significantly affect or alter the binding characteristics of the antibody containing the amino acid sequence. Conservative modifications can include amino acid substitutions, additions and deletions.

As used herein, the term "epitope" refers to a group of three dimensional structures on the antigen where the monoclonal antibody and fragments thereof would bind.

As used herein, the term "monoclonal" refers to an antibody that binds to a sequence of amino acids and has a single specific epitope on its target antigen.

Properdin is known to bind C3b with high affinity. It was discovered that the properdin binding to C5 and C5b is important for properdin functions and that monoclonal antibodies, which specifically inhibit properdin binding to C5 or C5b, inhibit properdin function via the inhibition of C5b-9 formation and inhibition of C3b formation. One embodiment of the present invention relates to the use of anti-properdin antibodies that can bind to properdin and inhibit properdin binding to C5 or C5b.

Properdin binds to C9 with high affinity, which suggests a role for properdin in MAC assembly. The antibodies used in the present invention can inhibit: a) AP activation and b) AP induced formation of C5b-9. The prevention of properdin binding to C5/C9 can allow for the inhibition of the alternative complement pathway in the mammal without affecting classical pathway activation. The ratio of binding of the antibody to properdin can be about 0.2:1 to about 1.5:1. The anti-properdin agent can prevent and/or reverse the binding of properdin to C5 and C9.

One embodiment of the present invention therefore relates to the use of anti-properdin antibodies that can bind to properdin and inhibit properdin binding to C5 or C5b required for its function. By inhibiting the binding of properdin to C5/C5b, the anti-properdin antibody can inhibit the formation of the C5b-9 complex, also known as MAC.

Properdin can also exist as a polymer, for example, a dimer, a trimer, or atetramer. Properdin binds to C3b, and antibodies that inhibit properdin binding to C3b have been developed. The present disclosure can include antibodies that can inhibit properdin binding to C5b and C9. C5 provides an anchor to the formation of C5b-9 complexes and C9 is responsible for the formation of part of the C5b-9 complex. The antibodies of the present disclosure can inhibit MAC assembly and provides a mechanism by which MAC is formed at the site of C3 convertase formation.

In one example, properdin can exist as a trimer. A trimer with three properdin molecule can include two sites that can be occupied by C5b. One site would be occupied by C3b as per the formula: (P)ₓ(C5b)_{y}(C3b)_{z}(Bb)_{w} in which _{X= Y+Z, Z = W.}. PC3bBb is a pure C3 convertase. P(C5b)C3bBb is also a convertase with C5b bound to it. This molecule can cleave C3 and C5, generating moles of C5b. As the pathway proceeds to making MAC, properdin- bound C3b could be replaced with C5b. Properdin bound C5b participates in the formation of C5b-9. Properdin also binds C9 with high affinity, which could further stabilize the formation of functional polymeric C9 molecule.

Another embodiment of the present invention relates to the use of antibodies that specifically bind to properdin, for example an anti-properdin antibody, to inhibit alternative complement pathway activation via the inhibition of properdin binding to C5 or C5b. The anti-properdin antibodies used in the present invention are directed to or specifically bind to domains on properdin that are involved in controlling properdin function. The anti-properdin antibodies or fragments thereof used in the present invention can inhibit AP activation without inhibiting or effecting the classical complement pathway by several methods including, but not limited to selectively inhibiting the binding of properdin to C5 or C5b, reducing the formation of membrane attack complex C5b-9, reducing the formation of anaphylatoxins, for example C3a and/or C5a, reducing the formation of C3b, reducing the activation of neutrophils, monocytes and platelets, and/or reducing leukocyte aggregate formation.

The amino acid sequences of mammalian properdin are known. For example, the amino acid sequence of human properdin is disclosed in the GenBank database under Accession No. AAA36489. Human properdin is a 469 amino acid protein that includes a signal peptide (amino acids 1-28), and six, non-identical thrombo spondin type 1 repeats (TSR) of about 60 amino acids each, as follows: amino acids 80-134 (TSR1), amino acids 139-191 (TSR2), amino acids 196-255 (TSR3), amino acids 260-313 (TSR4), amino acids 318-377 (TSR5), and amino acids 382-462 (TSR6).

Anti-properdin antibodies can be selected based on their ability to inhibit properdin binding to C5 and then selected based on AP dependent hemolysis of rRBC (rabbit erythrocytes). These antibodies can bind to properdin in about a 0.2:1 stoichiometric ratio. Inhibition of alternative pathway (AP) complement activation can cause reduced levels of C3a, C5a, and C5b-9, inhibit the activation of monocytes, inhibit the formation of monocyte-platelets, and inhibit neutrophil-platelet aggregates.

In another embodiment of the invention, the anti-properdin antibody can inhibit the binding of properdin to C5. In a further embodiment of the invention, the anti-properdin antibody can inhibit the binding of properdin to C5b. In yet another embodiment of the invention, the anti-properdin antibody can inhibit the binding of properdin to C9. In these embodiments, the anti-properdin antibody can affect the binding in about a 0.2:1 molar ratio of antibody to properdin, an about 1:1 molar ratio of antibody to properdin, an about 1.5:1 ratio of antibody to properdin and with a maximum of about 10:1 antibody to properdin.

In a further embodiment of the invention, the anti-properdin antibody can bind properdin with high affinity, can inhibit C5 binding, can inhibit C3a, C5a, and C5b-9 formation, can inhibit neutrophil, monocyte and platelet activation, can inhibit leukocyte platelet conjugate formation, and can prevent alternative complement pathway activation.

In yet another embodiment of the invention, the antibodies of the present invention can have no effect on classical pathway activation. Thus, the monoclonal antibodies of the present invention may not inhibit the classical pathway. Monoclonal antibodies used in the present invention can bind to a region on properdin that is only involved in AP activation via its binding to the C5 or C5b complement proteins.

In another embodiment of the invention, the antibodies used in the present invention can comprise heavy chains and light chains of CDR1, CDR2 and CDR3 of mAb, or combinations thereof. The amino acid sequences of the variable heavy chain are shown in SEQ ID NO: 1 (CDR1), SEQ ID NO: 2 (CDR2), and SEQ ID NO: 3 (CDR3). The variable light chain CDRs are shown SEQ ID NO: 4 (CDR1), SEQ ID NO: 5 (CDR2), and SEQ ID NO: 6 (CDR3). Accordingly, in another embodiment, the present disclosure can provide an isolated monoclonal antibody, or antigen binding portion thereof comprising the CDR regions that can bind to human properdin.

Accordingly, in yet another embodiment of the disclosure, the present disclosure can provide an isolated monoclonal antibody, or antigen binding portion thereof comprising: (a) a heavy chain variable region comprising of CDR1, CDR2, and CDR3 which include the amino acid sequences of SEQ ID NOs: 1, 2, and 3, respectively; and (b) a light chain variable region comprising CDR1, CDR2, and CDR3 which include the amino acid sequences of SEQ ID NOs: 4, 5, and 6, respectively; wherein the antibody specifically binds human properdin.

In another embodiment, an antibody of the present disclosure can comprise heavy and light chain variable regions comprising amino acid sequences that are homologous to the amino acid sequences of the preferred antibodies described herein, and wherein the antibodies retain the desired functional properties of the anti-properdin antibodies of the invention. For example, the disclosure can provide an isolated monoclonal antibody, or antigen binding portion thereof, comprising a heavy chain variable region and a light chain variable region, wherein: (a) the heavy chain variable region comprises an amino acid sequence that is at least 80% homologous to the amino acid sequence of SEQ ID NO: 1; (b) the light chain variable region comprises an amino acid sequence that is at least 80% homologous to the amino acid sequence of SEQ ID NO: 9; and (c) the antibody specifically binds to human properdin.

In various embodiments of the present invention, the anti-properdin antibody can be, for example, a humanized antibody or a chimeric antibody.

In yet other embodiments of the present invention, the V*_{H}* and/or V*_{L}* amino acid sequences of the antibodies of the present invention may be 85%, 90%, 95%, 96%, 97%, 98% or 99% homologous to the sequences set forth herein. An antibody having V*_{H}* and V*_{L}* regions having high homology, for example, 80% or greater, to the V*_{H}* and V*_{L}* regions of the sequences set forth above, can be obtained by mutagenesis.

In other embodiments, an antibody used in the present invention can include a heavy chain variable region comprising heavy chain CDR1, CDR2 and CDR3 sequences and a light chain variable region comprising of the light chain CDR1, CDR2 and CDR3 sequences, wherein one or more of these CDR sequences comprise specified amino acid sequences based on the preferred antibodies described herein, for example, NM4540, and wherein the antibodies retain the desired functional properties of the anti-properdin antibodies of the invention

In another embodiment of the invention, one example of an anti-properdin monoclonal antibody can be labeled as 14G11. The anti-properdin antibody 14G11 can bind properdin and can inhibit: properdin binding to C5 and C9, C3a, C5a, and C5b-9 formation, AP C3 convertase formation, and cellular activation. Properdin binds to C5 and can guide the formation of C5b-9 complex at the site of pathology. The anti-properdin agents used in the present invention can bind to properdin and inhibit the binding of properdin to C5 or C5b and, thus, can prevent AP activation.

The anti-properdin monoclonal antibody 14G11 used in the present invention can bind to the N-terminal domain of SEQ ID NO: 15. Such antibodies can inhibit the binding of properdin to C5b and thus can prevent AP activation.

The antibody used in the present invention can be a monoclonal, polyclonal, chimeric, recombinant, humanized, de-immunized, or Humaneered™ antibody. The antibody can also be an antibody fragment, such as a single chain antibody, IgG, F(ab')2, F(ab), F(ab') fragment, or truncated antibody (e.g., Fc truncated antibody). The antibody can be produced in a cell or mammal or it can be genetically engineered in a mammal, phage display or from a recombinant or natural antibody library. The antibody can be administered *in vivo* or *ex vivo.* The antibody can lack the ability to activate Fcγ receptors as well as lack immunogenicity in humans.

The administration of the neutralizing antibody used in the present invention or antigen binding fragment to the subject can result in at least one of the following: inhibition of properdin binding to C5, inhibition of properdin binding to C5b, inhibition of properdin binding to C9, reduction in the formation of C3b, reduction in the formation of C3bBb, reduction in the production of C3a and C5a, reduction in the formation of C5b-9 complex, reduction in the activation of neutrophils, reduction in the activation of monocytes reduction in the activation of platelets, reduction in the formation of leukocyte-platelet conjugates, reduction in TNF-alpha production, and the reduction in production of neutrophil elastase. Consequently, the antibody or fragment thereof can also inhibit the alternative pathway dependent rabbit erythrocyte lysis in human serum/plasma.

The humanized monoclonal anti-properdin antibodies listed in sequences SEQ ID NO: 7, SEQ ID NO: 8, SEQ ID NO: 9, SEQ ID NO: 10, SEQ ID NO: 11, SEQ ID NO: 12, SEQ ID NO: 13, and SEQ ID NO: 14 can contain at least one of the CDR sequences listed above. Additionally, the humanized monoclonal anti-properdin antibodies can contain any combination of at least one of the CDR sequences of the present invention.

### Therapeutic Antibodies

Currently accepted therapeutic antibodies comprise murine, chimeric, primatized, and humanized antibodies. Antigen binding fragments usually are considered single chain, variable region of the antibody, Fab, Fab2', truncated in the Fc domain, and scFv. Chimeric and humanized fragments derived from such proteins contain CDRs which are the main peptide segments for binding. Homologies in the CDRs across various antibodies that can bind the same target can range from 50-70%, 70-80%, and 80-95%, or 100% in some examples of the CDRs. These antibodies can be produced by various technologies such as CDR grafting, generating human antibodies in non-human primates, generating human antibodies in mouse, knockout mouse techniques as well as other methods can be employed to produce such antibodies.

Furthermore, human antibodies or antibodies from other species can be generated through display-type technologies, including, without limitation, phage display, retroviral display, ribosomal display, and other such techniques. The resulting molecules can be subjected to additional maturation, such as affinity maturation, as such techniques are well known.

The anti-properdin antibody or fragments thereof can be used in therapeutic methods for the prophylactic and therapeutic treatment of diseases mediated, directly or indirectly, by a component of the alternative complement pathway, and/or by a factor generated following activation of the alternative complement pathway, described herein.

### Additional Criteria for Antibody Therapeutics

The functional activity of the present invention can be measured by the ability of the monoclonal antibody to inhibit the alternative complement pathway. For example, an anti-properdin antibody used in the present invention can exhibit one or more of the following properties: (1) inhibits properdin binding to C5 (or C5b); (2) reduces PC3bBb formation/ or C3bBb formation, (3) reduces the concentration of free properdin, (4) reduces formation of C3b, (5) reduces formation of C3a, C5a and C5b-9, (6) reduces monocytes CD11b expression, (7) reduces neutrophil CD11b expression, (8) reduces platelet CD62 P expression, (9) reduces leukocyte-platelet conjugate formation, (10) reduces tumor necrosis factor alpha (TNF-α), and (11) reduces neutrophil elastase formation.

Bispecific antibodies, immunotoxins, radiolabeled therapeutics, generation of peptide therapeutics, gene therapies, particularly intrabodies, antisense therapeutics, and small molecules can be covered under this disclosure. Bispecific antibodies can be generated that comprise, for example, (i) two antibodies, one with a specificity to properdin and the other to a second molecule that are conjugated together, (ii) a single antibody that has one chain specific to properdin and a second chain specific to a second molecule, or (iii) a single chain antibody that has specificity to properdin and the other molecules. Such bi-specific antibodies can be generated using techniques that are well known in the art.

The anti-properdin antibody or fragments thereof used in the present invention can be used in therapeutic methods for the prophylactic and therapeutic treatment of diseases mediated, directly or indirectly, by a component of the alternative complement pathway, and/or by a factor generated following activation of the alternative complement pathway.

### Anti-Properdin Agents

In another embodiment, the present disclosure can provide methods of inhibiting the adverse effects of properdin-dependent complement activation. Anti-properdin inhibitory agents can be administered in an amount effective to inhibit properdin-dependent complement activation in a living subject. In the practice of this aspect of the disclosure, representative properdin inhibitory agents can include anti-properdin antibodies that can prevent binding of properdin to C5 and C5b, and can prevent production of C3a, C5a and C5b-9 by inhibition of the C5 association with properdin. The properdin inhibitory agents can be used alone as a primary therapy or in combination with other methods as complement to enhance the benefits of other treatments.

The inhibitory agents can be small molecules, aptamers, DNA fragments, small peptides representing CDR domains, and/or SiRNA. These inhibitory agents inhibit properdin binding to C5, C9, and C3b associated with C5.

### Aptamers

Aptamers can be produced that bind properdin with high affinity and are single-stranded oligonucleotides that fold in specific well-defined, three-dimensional shapes, which allow them to interact with a folded, three-dimensional protein target. Unlike monoclonal antibodies, aptamers have not been observed to be immunogenic. Since nucleic acids are not typically recognized by the human immune system as foreign agents, aptamers do not generally trigger an antibody response. The three-dimensional structure of aptamers creates a large surface area of interaction between an aptamer and its target protein, making aptamers ideally suited for blocking protein-protein interactions. Furthermore, since aptamers interact with proteins found on the surface of and outside cells, aptamers do not have to cross the cell membrane, which can make it easier to deliver an effective quantity of aptamer to the target. Aptamers are chemically stable and can be designed to resist metabolic degradation in the human body. Using standard, defined chemical modifications and conjugations, aptamers can be specifically engineered to have an optimal half-life, or the ability to maintain effective concentration. Aptamers are chemically synthesized allowing for rapid, scalable and reproducible production at lower costs.

### Anti-Properdin Antibodies

In the use of the present invention, the properdin inhibitory agent comprises an anti-properdin antibody that selectively inhibits the alternative pathway by inhibiting properdin binding to C5 and C5b. Such antibodies can include polyclonal, monoclonal or recombinant antibodies derived from any antibody-producing mammal and may be multispecific, chimeric, humanized, anti-idiotype, and antibody fragments. Antibody fragments can include Fab, Fab', F(ab)₂, F(ab')₂, Fv fragments, scFv fragments and single-chain antibodies as further described herein. The antibodies described in this invention are those that can inhibit properdin binding to C5, C5b and C9.

### Production of Anti-Properdin Antibodies

Production of anti properdin antibodies can be completed by utilizing intact properdin, properdin polypeptides, and/or using antigenic properdin epitope-bearing peptides, for example, a portion of the properdin polypeptide. It is possible to isolate the properdin peptides and polypeptides used to raise antibodies as natural polypeptides, recombinant or synthetic peptides, and catalytically inactive recombinant polypeptides. This embodiment of the present disclosure can provide anti-properdin antibodies obtained using a transgenic mouse strain. Antigens useful for producing anti properdin antibodies also include fusion polypeptides.

### Polyclonal Antibodies

Using well-known methods, polyclonal antibodies against properdin can be prepared by immunizing an animal with properdin polypeptide or an immunogenic portion thereof to generate polyclonal antibodies. Polyclonal antibodies are typically raised in animals such as dogs, goats, chickens, rats, mice, horses, cows, rabbits, guinea pigs, or sheep. Alternatively, an anti-properdin antibody used in the present invention can also be derived from a subhuman primate and/or produced by technologies sold by epitomics or by cell DNA/RNA methodologies. Sera containing immunologically active antibodies are then produced from the blood of such immunized animals using standard procedures.

### Monoclonal Antibodies

In some embodiments of the use of the present invention, the properdin inhibitory agent can be a highly specific anti-properdin monoclonal antibody being directed against an epitope. As used herein, the modifier "monoclonal" indicates the character of the antibody as being obtained from a substantially homogenous population of antibodies. Monoclonal antibodies can be obtained using any technique that provides for the production of antibody molecules by continuous cell lines in culture, such as a hybridoma method.

Human monoclonal antibodies can be obtained using transgenic mice that have been engineered to produce specific human antibodies in response to antigenic challenge.

In one embodiment of the invention, the anti-properdin monoclonal antibodies useful for the use of the present invention can bind to properdin with a binding affinity of <100 pM, preferably <10 pM and most preferably <2 pM.

### Chimeric/Humanized Antibodies

Chimeric antibodies are humanized forms of non-human for example, murine, antibodies, which contain minimal sequence derived from non-human immunoglobulin. Humanized monoclonal antibodies are produced by transferring the non-human animal, for example, a mouse, CDRs from the heavy and light variable chains of the mouse immunoglobulin into a human variable domain. Typically, residues of human antibodies are then substituted in the framework regions of the non-human counterparts. The humanized antibodies of the present invention can include properdin binding to a CDR3 region. In addition, the Fc portions of the anti-properdin antibody used in the present invention can be replaced to produce IgA or IgM as well as human IgG antibodies.

### Recombinant Antibodies

Anti-properdin antibodies can also be made using recombinant methods. For example, human antibodies can be made using human immunoglobulin expression libraries (available for example, from Stratagene, Corp., La Jolla, Calif or Bioatla, San Diego, CA) to produce fragments of human antibodies (V_{H}, V_{L}, Fv, Fd, Fab or F(ab')₂), which are then used to construct whole human antibodies using techniques similar to those for producing chimeric antibodies.

### Immunoglobulin Fragments

The Properdin inhibitory agents used in the present invention can encompass intact immunoglobulin molecules as well as the relatively well known fragments including Fab, Fab', F(ab).sub.2, F(ab').sub.2 and Fv fragments, scFv fragments, diabodies, linear antibodies, single-chain antibody molecules and multispecific antibodies formed from antibody fragments.

Proteolytic hydrolysis, such as by pepsin or papain digestion of whole antibodies by conventional methods, can obtain antibody fragments. For example, antibody fragments can be produced by enzymatic cleavage of antibodies with pepsin to provide F(ab')₂. This fragment can be further cleaved using a thiol reducing agent to produce Fab' monovalent fragments. In another example, the cleavage reaction can be performed using a blocking group for the sulfhydryl groups that result from cleavage of disulfide linkages. In yet another example, an enzymatic cleavage using pepsin produces two monovalent Fab fragments and an Fc fragment directly.

In some embodiments of the present invention, antibody fragments lacking the Fc region can be preferably used to avoid activation of the classical complement pathway, which is initiated upon binding Fc to the Fcγ receptor. One can produce a MoAb that avoids Fcγ receptor interactions by several methods. For example, the Fc region of a monoclonal antibody can be removed chemically using partial digestion by proteolytic enzymes (such as ficin digestion), thereby generating, for example, antigen-binding antibody fragments such as Fab or F(ab)₂. In another example, the human γ4 IgG isotype, which does not bind Fcγ receptors, can be used during the construction of a humanized antibody as described herein. Antibodies, single chain antibodies and antigen-binding domains, that lack the Fc domain can also be engineered using recombinant techniques described herein.

In addition, a single peptide chain can be created that can bind molecules specific for properdin in which the heavy and light chain Fv regions are connected. The Fv fragments can be connected by a peptide linker to form a single chain antigen binding protein (scFv).

### Additional Agents

The compositions and methods comprising properdin inhibitory agents can comprise one or more additional therapeutic agents. These additional agents can augment the activity of the properdin inhibitory agent or that provide related therapeutic functions in an additive or synergistic fashion. For example, one or more properdin inhibitory agents can be administered in combination with one or more anti-inflammatory and/or analgesic agents.

### Preparation of Antibodies

Antibodies in accordance with the present invention can be prepared in an animal, for example a mouse, using standard methods well known in the art. The monoclonal antibody used in the present invention can be converted into a humanized version for therapeutic use. Fully human antibodies can also be generated by methods well known in art. The antibody can be made into a humanized, fully human, humaneered, chimeric, recombinant either by contract or in house for therapeutic applications. Cell lines of particular preference are selected through determining which cell lines have high expression levels and produce antibodies with constitutive properdin binding properties.

The antibodies used in the present invention can also be generated using properdin knockout rodents, for example, mice, to generate effective blocking antibodies. Since these rodents do not contain the properdin gene, the rodents would see the injection of properdin as an immunogenic antigen, rather than a normal protein present in the system. Based on this recognition of the injected properdin as an antigen, the rodent will generate antibodies against it. The benefit of using a knockout rodent allows for increased probability for the generation of an antibody that is anti-rat, anti-mouse and anti-any other species, depending on the rodent being used.

Antibodies can be expressed as tetramers containing two light and two heavy chains, as separate heavy chains, light chains, as Fab, Fab', F(ab')2, and Fv, or as single chain antibodies in which heavy and light chain variable domains are linked through a spacer.

For some applications, non-IgG antibodies can be useful. For example, where multivalent antibody complexes are desired, IgM and IgA antibodies can be used. Chimeric, humanized and human antibodies are typically produced by recombinant expression.

Alternatively, antibody coding sequences can be incorporated in transgenes for introduction into the genome of a transgenic animal and subsequent expression in the transgenic animal with methods well known in the art.

The recombinant antibody can be of the murine class IgG₁, IgG₂ₐ, IgG_{2b}, IgM, IgA, IgD or IgE, the human classes IgG₁, IgG₂, IgG₃, IgG₄, IgM, IgA₁, IgA₂, IgD or IgE, or combinations or fragments thereof. In one example, the chimeric antibody library is composed of primarily IgG antibodies or Fab antibody fragments.

### Treatment Methods

The methods of the present disclosure can generally involve the steps of: administering to a mammalian subject in need thereof of an effective amount or therapeutically effective amount of a subject antibody for methods of reducing the level of a polypeptide generated following activation of the alternative complement pathway; reducing the level of C5b-9 or membrane attack complex (MAC); reducing the level of an anaphylatoxin; methods of reducing the level of newly formed C3b, P, Bb, and C5b-9; and treating a disease or disorder mediated by the alternative complement pathway.

An "effective amount" or "therapeutically effective amount" of a subject antibody is an amount that is effective to reduce the production and/or level of a polypeptide generated following activation of the alternative complement pathway by at least about 20%, at least about 30%, at least about 40%, at least about 50%, at least about 60%, at least about 70%, at least about 80%, at least about 90%, or more.

The anti-properdin antibody can be administered to an individual in a formulation with a pharmaceutically acceptable excipient(s). A wide variety of pharmaceutically acceptable excipients is known in the art and need not be discussed in detail herein.

In the methods provided by the present disclosure, a subject antibody can be administered to the host using any convenient means capable of resulting in the desired therapeutic effect. Thus, the antibody can be incorporated into a variety of formulations for therapeutic administration. In one example, a subject antibody can be formulated into pharmaceutical compositions by combination with appropriate, pharmaceutically acceptable carriers or diluents, and can be formulated into preparations in solid, semi-solid, liquid or gaseous forms, such as tablets, capsules, powders, granules, ointments, solutions, suppositories, injections, inhalants and aerosols.

As such, administration of a subject antibody can be achieved in various administrations, including oral, buccal, rectal, parenteral, intraperitoneal, intradermal, subcutaneous, intramuscular, transdermal, intranasal, pulmonary, intratracheal, etc administrations.

In pharmaceutical dosage forms, the agents can be administered in the form of their pharmaceutically acceptable salts, or they can also be administered independently or in appropriate association, as well as in combination, with other pharmaceutically active compounds. The following methods and excipients are merely exemplary and are in no way limiting.

Unit dosage forms for injection or intravenous administration can comprise the inhibitor(s) in a composition as a solution in sterile water, normal saline or another pharmaceutically acceptable carrier.

An antibody used in the present invention can be administered to an individual with a certain frequency and for a period of time so as to achieve the desired therapeutic effect. For example, an antibody used in the present invention can be administered, for example, once per month, twice per month, three times per month, every other week (qow), once per week (qw), twice per week (biw), three times per week (tiw), four times per week, five times per week, six times per week, every other day (qod), daily (qd), twice a day (qid), or three times a day (tid), or substantially continuously, or continuously, over a period of time ranging from about one day to about one week, from about two weeks to about four weeks, from about one month to about two months, from about two months to about four months, from about four months to about six months, or longer.

### Combination Therapy

The anti-properdin antibody can, in some embodiments, be administered in an effective amount in combination therapy with a second therapeutic agent.

### Disease Conditions

In another aspect of the invention, the antibodies of the present invention can be used to inhibit complement activation via the alternative pathway *in vivo* in subjects, including humans, suffering from an acute or chronic pathological injury. The present invention can be used in conjunction with the following diseases, disorders, injuries, and treatments, including but not limited to:

Extracorporeal circulation diseases and disorders: Post-cardiopulmonary bypass inflammation, post-operative pulmonary dysfunction, cardiopulmonary bypass, hemodialysis, leukopheresis, plasmapheresis, plateletpheresis, heparin-induced extracorporeal LDL precipitation (HELP), postperfusion syndrome, extracorporeal membrane oxygenation (ECMO), cardiopulmonary bypass (CPB), post-perfusion syndrome, systemic inflammatory response, and multiple organ failure.

Cardiovascular diseases and disorders: acute coronary syndromes, Kawaski disease (arteritis), Takayasu's arteritis, Henoch-Schonlein purpura nephritis, vascular leakage syndrome, percutaneous coronary intervention (PCI), myocardial infarction, ischemia-reperfusion injury following acute myocardial infarction, atherosclerosis, vasculitis, immune complex vasculitis, vasculitis associated with rheumatoid arthritis (also called malignant rheumatoid arthritis), systemic lupus erythematosus-associated vasculitis, sepsis, arteritis, aneurysm, cardiomyopathy, dilated cardiomyopathy, cardiac surgery, peripheral vascular conditions, renovascular conditions, cardiovascular conditions, cerebrovascular conditions, mesenteric/enteric vascular conditions, diabetic angiopathy, venous gas embolus (VGE), Wegener's granulomatosis, heparin-induced extracorporeal membrane oxygenation, and Behcet's syndrome.

Bone/Musculoskeletal diseases and disorders: arthritis, inflammatory arthritis, non-inflammatory arthritis, rheumatoid arthritis, juvenile rheumatoid arthritis, systemic juvenile rheumatoid arthritis, osteoarthritis, osteoporosis, systemic lupus erythematosus (SLE), Behcet's syndrome, and Sjogren's syndrome.

Transplantation diseases and disorders: transplant rejection, xenograft rejection, graft versus host disease, xenotransplantation of organs or grafts, allotransplantation of organs or grafts, and hyperacute rejection.

Eye/Ocular diseases and disorders: wet and dry age-related macular degeneration (AMD), choroidal neurovascularization (CNV), retinal damage, diabetic retinopathy, diabetic retinal microangiopathy, histoplasmosis of the eye, uveitis, diabetic macular edema, diabetic retinopathy, diabetic retinal microangiopathy, pathological myopia, central retinal vein occlusion (CRVO), corneal neovascularization, retinal neovascularization, retinal pigment epithelium (RPE), histoplasmosis of the eye, and Purtscher's retinopathy.

Hemolytic/Blood diseases and disorders: sepsis, systemic inflammatory response syndrome" (SIRS), hemorrhagic shock, acute respiratory distress syndrome (ARDS), catastrophic anti-phospholipid syndrome (CAPS), cold agglutinin disease (CAD), autoimmune thrombotic thrombocytopenic purpura (TTP), endotoxemia, hemolytic uremic syndrome (HUS), atypical hemolytic uremic syndrome (aHUS), paroxysmal nocturnal hemoglobinuria (PNH), sepsis, septic shock, sickle cell anemia, hemolytic anemia, hypereosinophilic syndrome, and anti-phospholipid syndrome (APLS).

Respiratory/Pulmonary diseases and disorders: asthma, Wegener's granulomatosis, transfusion-related acute lung injury (TRALI), antiglomerular basement membrane disease (Goodpasture's disease), eosinophilic pneumonia, hypersensitivity pneumonia, allergic bronchitis bronchiecstasis, reactive airway disease syndrome, respiratory syncytial virus (RSV) infection, parainfluenza virus infection, rhinovirus infection, adenovirus infection, allergic bronchopulmonary aspergillosis (ABPA), tuberculosis, parasitic lung disease, adult respiratory distress syndrome, chronic obstructive pulmonary disease (COPD), sarcoidosis, emphysema, bronchitis, cystic fibrosis, interstitial lung disease, acute respiratory distress syndrome (ARDS), transfusion-related acute lung injury, ischemia/reperfusion acute lung injury, byssinosis, heparin-induced extracorporeal membrane oxygenation, anaphylactic shock, and asbestos-induced inflammation.

Central and Peripheral Nervous System/Neurological diseases and disorders: multiple sclerosis (MS), myasthenia gravis (MG), Guillain Barre syndrome, Miller-Fisher syndrome, stroke, reperfusion following stroke, Alzheimer's disease, multifocal motor neuropathy (MMN), demyelination, Huntington's disease, amyotrophic lateral sclerosis (ALS), Parkinson's disease, degenerative disc disease (DDD), meningitis, cranial nerve damage from meningitis, variant Creutzfeldt-Jakob Disease (vCJD), idiopathic polyneuropathy, brain/cerebral trauma (including, but not limited to, hemorrhage, inflammation, and edema), and neuropathic pain.

Trauma-induced injuries and disorders: hemorrhagic shock, hypovolemic shock, spinal cord injury, neuronal injury, cerebral trauma, cerebral ischemia reperfusion, crush injury, wound healing, severe burns, and frostbite.

Renal diseases and disorders: renal reperfusion injury, poststreptococcal glomerulonephritis (PSGN), Goodpasture's disease, membranous nephritis, Berger's Disease/IgA nephropathy, mesangioproliferative glomerulonephritis, membranous glomerulonephritis, membranoproliferative glomerulonephritis (mesangiocapillary glomerulonephritis), acute postinfectious glomerulonephritis, cryoglobulinemic glomerulonephritis, lupus nephritis, Henoch-Schonlein purpura nephritis, and renal cortical necrosis (RCN).

Reperfusion injuries and disorders of organs: including but not limited to heart, brain, kidney, and liver.

Reproduction and urogenital diseases and disorders: painful bladder diseases and disorders, sensory bladder diseases and disorders, spontaneous abortion, male and female diseases from infertility, diseases from pregnancy, fetomaternal tolerance, pre-eclampsia, urogenital inflammatory diseases, diseases and disorders from placental dysfunction, diseases and disorders from miscarriage, chronic abacterial cystitis, and interstitial cystitis.

Skin/Dermatologic diseases and disorders: burn injuries, psoriasis, atopic dermatitis (AD), eosinophilic spongiosis, urticaria, thermal injuries, pemphigoid, epidermolysis bullosa acquisita, autoimmune bullous dermatoses, bullous pemphigoid, scleroderma, angioedema, hereditary angioneurotic edema (HAE), erythema multiforme, herpes gestationis, Sjogren's syndrome, dermatomyositis, and dermatitis herpetiformis.

Gastrointestinal diseases and disorders: Crohn's disease, Celiac Disease/ gluten-sensitive enteropathy, Whipple's disease, intestinal ischemia, inflammatory bowel disease, and ulcerative colitis.

Endocrine diseases and disorders: Hashimoto's thyroiditis, juvenile lymphocytic thyroiditis, stress anxiety, and other diseases affecting prolactin, growth or insulin-like growth factor, adrenocorticotropin release, pancreatitis, Addison's disease, diabetic conditions including, but not limited to, type 1 and type 2 diabetes, type I diabetes mellitus, sarcoidosis, diabetic retinal microangiopathy, non-obese diabetes (IDDM), angiopathy, neuropathy or retinopathy complications of IDDM or Type-2 diabetes, and insulin resistance.

Treatment of Malignancies: diseases and disorders arising from chemotherapeutics and radiation therapy.

The foregoing description and Examples detail certain preferred embodiments of the invention and describe the best modes of usage as contemplated by the inventors. It will be appreciated, however, that no matter how detailed the foregoing may appear in text, the invention may be practiced in many ways and the invention should be construed in accordance with the appended claims and any equivalents thereof.

### EXAMPLES

Unless stated otherwise all reagents were of highest grade available. All complement proteins, alternative and classical pathway buffers, detection antibodies, and erythrocytes were from Complement Technologies (Tyler, TX) or Quidel Corporation (San Diego, CA). Flow cytometry antibodies were from BD Biosciences, San Jose, CA.TMB substrate was from Kirkegaard & Perry Limited, Gaithersberg, MD. All secondary antibodies were from American Qualex, San Clemente, CA, BSA and other reagents were all from Sigma-Aldrich, St Louise, MO.

ELISA plate readers (SpectraMax 190 and 250) were from Molecular Devices, and Flow Cytometer was FACS Calibur. Varity 3D program was used for data analyses, Curve fittings were done using MicroCal Origin program. Hemolysis kinetic assay was run using SectraMax, Molecular Devices., ELISA plates were from Corning Costar, Lowell, MA.

Anti-properdin antibodies that were generated at NovelMed Thrapeutics and the corresponding cell lines have been stored at the ATCC. F(ab)2 and Fab were generated using appropriate enzymatic methods and verified for purity using gel electrophoresis.

### Example 1

### Properdin binds to complement proteins C3b, C5/C5b, and C9

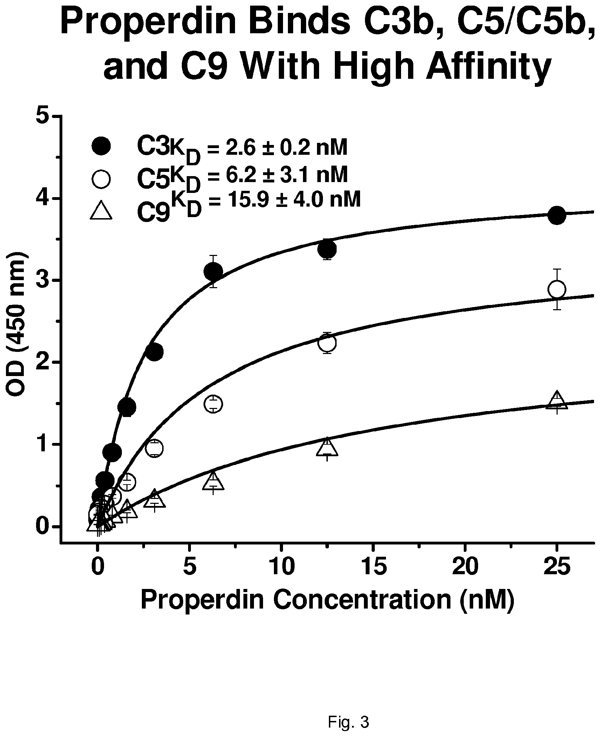

Properdin is known to bind C3b with high affinity. We demonstrate that properdin binds C5/C5b and C9 with high affinity and a minimal or no binding of properdin to C6, C7, and C8. Both C3 and C5 exist as intact proteins, however, when substrate-bound, they are converted into C3b and C5b, respectively. No published reports thus far have demonstrtaed the binding of properdin to C5 and or C5b. By inhibiting the binding of properdin to C5/C5b, we demonstrate that activation of the alternative pathway is blocked. Polystyrene microtiter plates were coated with human C3, C5, C6, C7, C8, and C9 at 2.0 µg/50 µl per well in phosphate buffered saline (PBS) overnight at 4°C. After aspirating the protein solution, the wells were blocked with PBS containing 1% bovine serum albumin (BSA) for 2 hours at room temperature. Wells without complement protein coating served as background controls. Aliquots of the properdin at varying concentration in blocking solution were added and plates were allowed to incubate for 1 hour. Properdin bound to the C3b, C5/C5b, C6, C7, C8, and C9 was measured by adding detection antibody goat anti-human properdin polyclonal antibody at 1:2000 dilutions in blocking solution. The plate was allowed to incubate for 1 hour at room temperature. After washing the plates with PBS, a peroxidase-conjugated rabbit anti-goat antibody (1:2000 dilution in blocking solution) was added and allowed to incubate for 1 hour. The plate was again rinsed thoroughly with PBS, and 100 µl of 3,3',5,5'-tetramethyl benzidine (TMB) substrate was added. After incubation for 30 minutes at 25°C, the reaction of TMB was quenched by the addition of 100 µl of phosphoric acid, and the plate was read at 450 nm in a microplate reader. As shown in Fig. 3, properdin binds to substrate bound C3b, C5/C5b, and C9 with high affinity values of 2.6, 6.2, and 15.9 nM, respectively. No or minimal binding was observed with C6, C7, and C8 proteins.

### Example 2

### Anti-properdin IgG and F(ab')₂ Inhibit alternative pathway (AP) dependent rabbit red blood cell (rRBC) Lysis

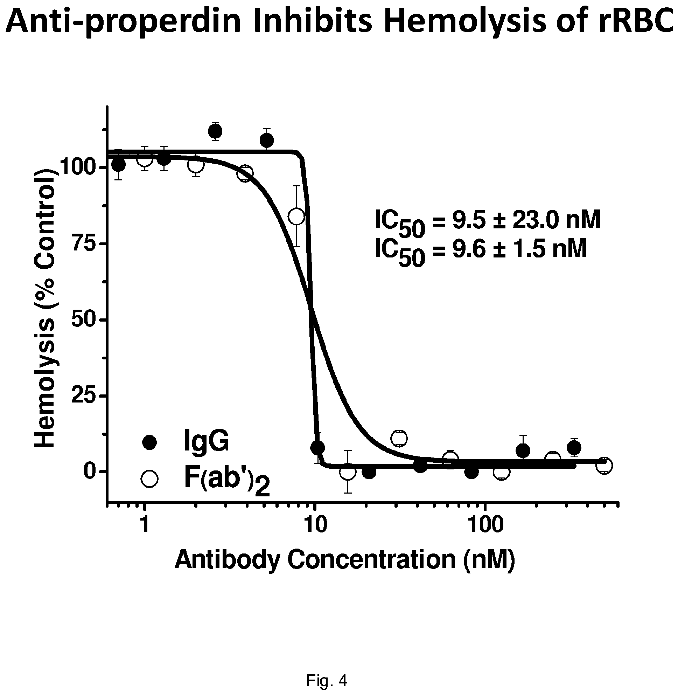

This erythrocyte lysis assay is based on the formation of a terminal complement complex on the surface of the rRBC. As a result, the rRBCs are lysed. The progressive decrease in light scatter at 700 nm is a direct measure of erythrocyte lysis. Typically, rRBC(s) are incubated in normal human serum in gelatin veronal buffer containing 5mM MgCl₂. Under these conditions, the surface of rRBC triggers the activation of the alternative pathway in normal human serum. The alternative pathway activation leads to the formation of C5b-9 complex on the surface of the rRBC(s). Agents that inhibit the formation of C5b-9 complexes are expected to inhibit cellular lysis. To evaluate the effect of anti-properdin antibody and fragments thereof, various concentrations of IgG, F(ab')₂, and Fab were incubated with normal human serum (10% NHS) in AP buffer at 37°C with a fixed concentration of rabbit erythrocytes in a temperature controlled ELISA plate reader capable of reading at 700 nm. A progressive decrease in light scatter (due to lysis of intact cells) was measured at 700 nm as a function of time. The data were recorded and analyzed with a SpectraMax 190 plate reader and SoftMax software. Total inhibition was calculated at each concentration of the IgG, F(ab')2, and fab and the results were expressed as a percent of unlysed controls. Data at each concentration was plotted in a sigmoidal plot with MicroCal Origin Software. As shown in Fig.4, IgG and F(ab)₂ inhibit AP dependent hemolysis of rRBC in normal human serum with an IC₅₀ of 10 nM inhibiting erythrocyte lysis. The antibodies are able to inhibit lysis with an IC₅₀ of approximately 10 nM.

### Anti-properdin monoclonal antibodies do not inhibit classical pathway activation

Monoclonal antibodies used in the present invention do not inhibit the classical pathway, which is required for proper function of host defense. Antibody sensitized sheep erythrocytes were incubated with 1% or 10% normal human serum in gelatin veronal buffer containing calcium (5 mM CaCl₂/MgCl₂) buffer. Antibody sensitized sheep cells activate the classical pathway. As a result, C5b-9 is formed on the surface of the erythrocyte and causes cell lysis. We tested the antibody in 1% and 10% normal human serum. Under both conditions, NM9401 inhibited erythrocyte lysis. In a typical assay, erythrocytes are incubated in 1%/10% normal human serum in CP buffer to allow complement activation to occur. As a result of CP activation, C5b-9 is formed on the surface of erythrocytes causing cellular lysis. The progressive decrease in light scattering due to cellular lysis is measured at 700 nm as a function of time. The anti properdin IgG does not inhibit the lysis of the antibody sensitized sheep cells at 1% and 10% serum concentration. The no serum control showed no effect on cell lysis, as expected. These results suggest that anti-properdin antibodies are capable of selectively inhibiting the alternative complement pathway without affecting the classical pathway activation.

### Example 3

### Anti-properdin IgG and F(ab')2 Bind Human Properdin with High Affinity

The affinity of anti-properdin IgG and F(ab)2 to human properdin is in the range of low pM range. The antibody and its fragments bind properdin with similar affinities.

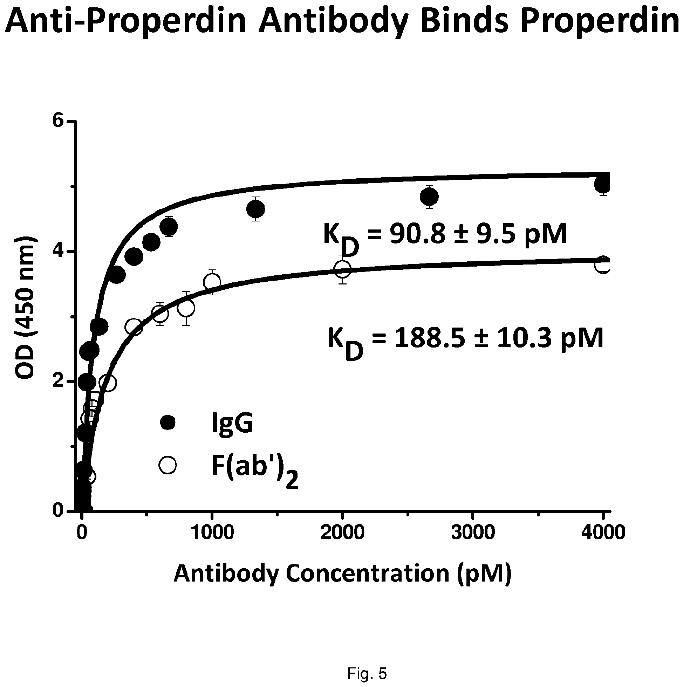

Polystyrene microtiter plates were coated with human (2.0 µg/50 µl per well) properdin in phosphate buffered saline (PBS) overnight at 4°C. After aspirating the properdin solution, the wells were blocked with PBS containing 1% bovine serum albumin (BSA) (Sigma-Aldrich, St. Louis, Mo.) for 1 hour at room temperature. Wells without peptide or properdin coating served as background controls. Aliquots of monoclonal anti-properdin antibody IgG, Fab2, and Fab in blocking solution were added to the properdin coated wells and allowed to incubate for 1 hour to allow for binding to occur. Following the 1 hour incubation at room temperature, the plate was rinsed with PBS five times and incubated with 1:2000 diluted detection peroxidase-conjugated goat anti-mouse monoclonal antibody. Following this incubation, the plate was rinsed and the bound peroxidase was identified using TMB reagent. As shown in Fig.5, Both, IgG and F(ab)₂ bind properdin with high affinity.

### Example 5

### Anti-properdin Fab2 inhibits properdin interaction with C5/C5b and C9

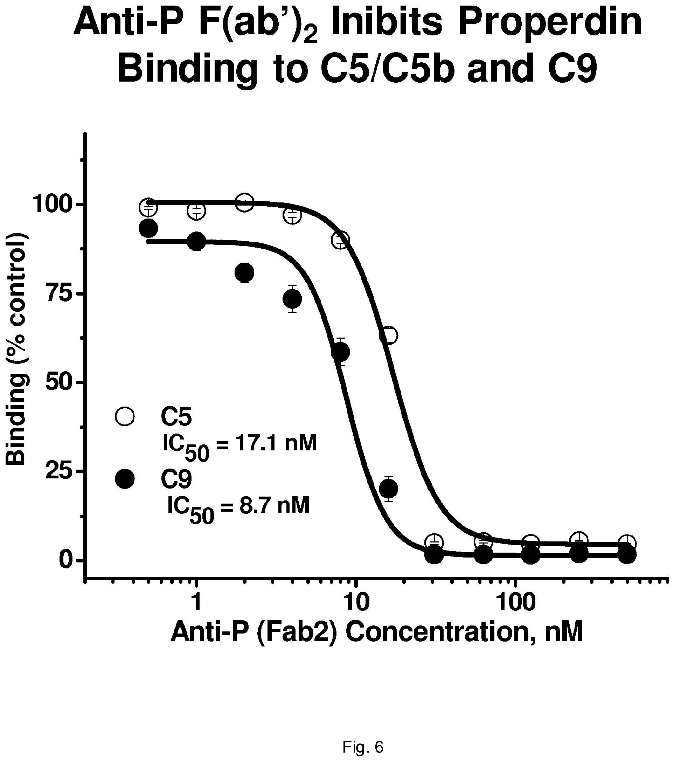

We demonstrated that properdin binds C5/C5b and C9 with high affinity. In this experiment we demonstrate that anti-properdin F(ab')₂ inhibits properdin interaction with C5/C5b and C9 with an affinity of 8 to 20 nM. Polystyrene microtiter plates were coated with human C5 and C9 at 2.0 µg/50 µl per well in phosphate buffered saline (PBS) overnight at 4°C. After aspirating the protein solution, the wells were blocked with PBS containing 1% bovine serum albumin (BSA) for 2 hours at room temperature. Wells without complement protein coating served as background controls. Aliquots of properdin at 50 nM were incubated with various concentrations of anti-properdin F(ab')₂ in blocking solution onto C5 and C9 coated plates and these plates were allowed to incubate for 1 hour at room temperature. Properdin bound to C5/C5b and C9 was measured by adding detection antibody goat anti-human properdin polyclonal antibody at 1:2000 dilutions in blocking solution. The plate was allowed to incubate for 1 hour at room temperature. After washing the plates with PBS, a peroxidase-conjugated rabbit anti-goat antibody (1:2000 dilution in blocking solution) was added and allowed to incubate for 1 hour. The plate was again rinsed thoroughly with PBS, and 100 µl of 3,3',5,5'-tetramethyl benzidine (TMB) substrate was added. After incubation for 30 minutes at 25°C., the reaction of TMB was quenched by the addition of 100 µl of phosphoric acid, and the plate was read at 450 nm in a microplate reader. As shown in Fig. 6, anti-properdin Fab₂ inhibits properdin interaction with C5 and C9, suggesting that properdin interaction with C5 and C9 are important for the inhibition of the alternative pathway. Complete inhibition was observed at around 20 nM of anti-properdin suggesting the ratio of 0.2:1 for antibody to properdin.

### Example 6

Anti properdin IgG and fragments thereof inhibit the formation of C3 convertase (PC3bBb) of alternative complement pathway as measured by the deposition of properdin, C3b, Bb, C9, C9, and C5b-9.

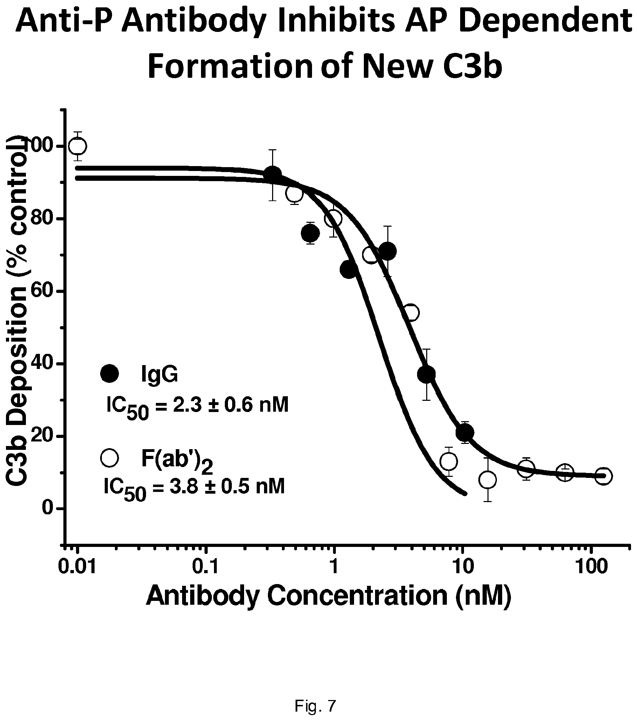

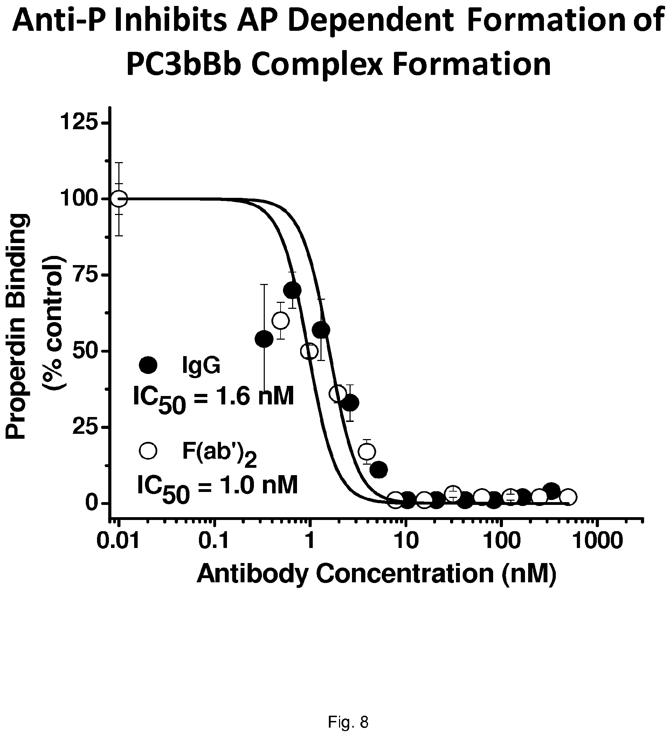

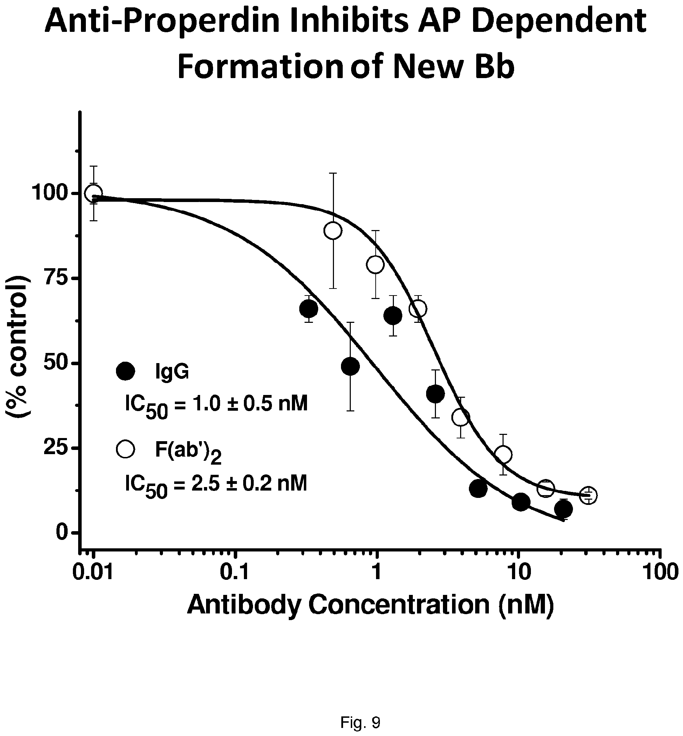

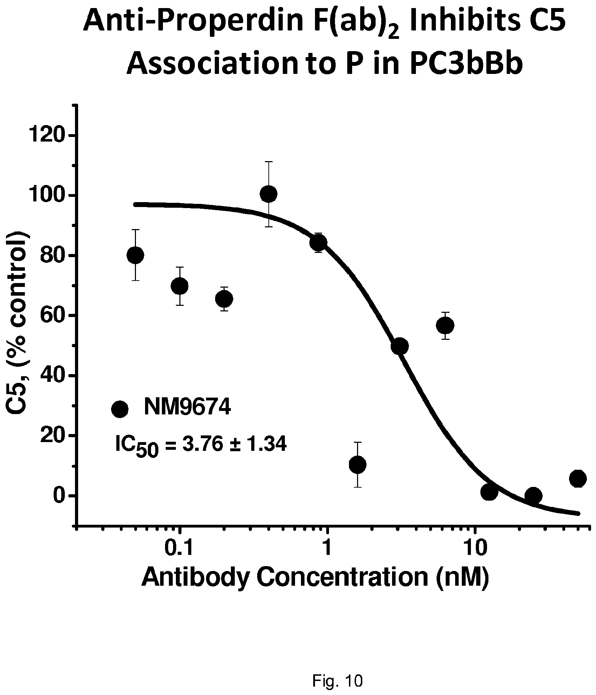

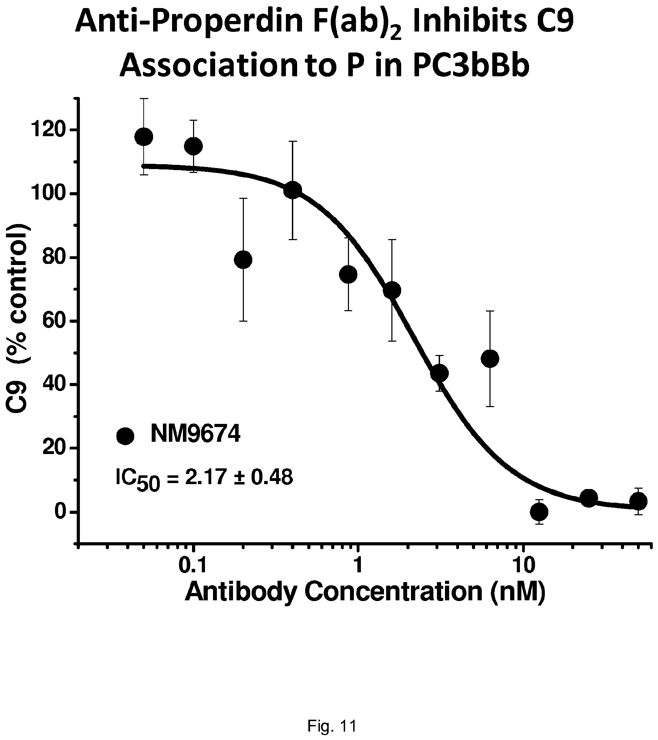

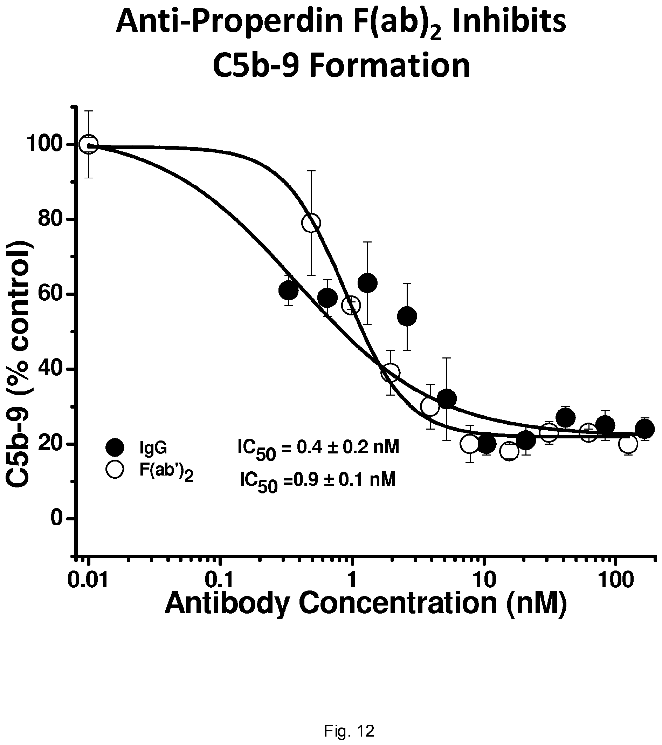

Alternative complement pathway is activated in Normal human serum by lipopolysaccharide from *Salmonella Typhosa.* We have utilized this paradigm to demonstrate whether anti-properdin antibody used in this invention would inhibit the formation of PC3bBb. We measured the deposition of factor P, C3b, Bb, C5b, C9, and C5b-9 in the presence and absence the anti-properdin antibody and fragments thereof. The formation of C3 and C5 convertases were detected with appropriate antibodies. In the presence of anti-properdin antibodies, a dose dependent inhibition of C3 and C5 convertase formation was noticed. In a typical assay, polystyrene microtiter plate wells were coated with LPS (Lipopolysaccharide from *Salmonella Typhosa*) at 2 µg/50 µl in PBS overnight. The wells were incubated with 1% BSA in PBS to block the unoccupied sites in the wells. Following a 2-hour blocking at room temperature and rinsing with PBS, normal human serum (10%) in AP buffer was mixed with varying concentrations of the antibody and fragments thereof. The mixture was incubated onto LPS coated wells. The plate was incubated for 2 hours 37°C to allow complement AP activation to occur. Following incubation, the plates were extensively washed with PBS, and components of the C3 convertase were detected with the appropriate antibodies. We detected C3b with rabbit anti-human C3c at 1:2000 in blocking solution, goat anti-human P at 1:2000 in blocking solution and goat anti-human factor Bb at 1:500 in blocking solution and HRPO conjugated anti-human C5b-9 at 1:2000 in blocking solution. Plates were incubated with their respective antibodies for 1-hour at room temperature. Following the incubation, the plates were rinsed with PBS and the bound antibodies were detected with peroxidase labeled goat anti-rabbit at 1:2000 for C3b and peroxidase labeled rabbit anti-goat at 1:2000 in blocking solution for properdin detection. All plates were developed with TMB following extensive washing with PBS. The blue color was quenched with 1 M ortho-phosphoric acid. The presence of C3b (Figure 7), factor P (Figure 8), Bb (Figure 9), C5b (Figure 10), and C9 (Figure 11) and MAC (Figure 12) together are indicative of AP C3 convertase formation. Fig. 8 demonstrates a dose dependent inhibition of factor P deposition, Fig. 9 demonstrates a dose dependent deposition of Bb, Fig. 7 demonstrates a dose dependent deposition of C3b formation, and Fig. 12 demonstrates a dose dependent deposition of C5b-9 deposition by anti-properdin antibodies. These data provide direct evidence that anti-properdin monoclonal antibodies prevented convertase formation and inhibited AP activation.

### Example 7

### DNA cloning and sequencing of a murine monoclonal antibody designated anti properdin14G11

The immunoglobulin specific mRNAs contained in cell line 14G11 were analyzed by RT-PCR and DNA sequencing. The results demonstrate that the cell line transcribed an IgG1 Kappa antibody. In addition to the major transcripts, two variant non-translated kappa transcripts (pseudogenes) were obtained. RT-PCR - cDNA was created from the RNA by reverse-transcription with an oligo(dT) primer. PCR reactions were set up to isolate and amplify both the variable and constant heavy and light regions of the cell line and utilized oligo-dT primers for the constant heavy regions and full-length light chains and specific primers for the variable heavy and variable light regions. The cDNA reactions were tailed with TdT in order to permit an oligo dT specific primer to be used to generate fully-extended (at the 5' end) clones. The purified inserts were cloned into pBluescript and the clones were picked from each reaction. Miniprep DNA was isolated, restriction mapped and the positive clones were analyzed through sequencing. The resulting sequencing data can be seen below.

### Results of Sequencing of NM14G11.9

### 14G11.9 Heavy Chain Results - Nucleotide sequence of Heavy chain variable region NM14G11.9

### Amino Acid Sequence of NovelMed Heavy chain variable region NM14G11.9

MGRLTSSFLLLIVPAYVLSQVTLKESGPGILQPSQTLSLTCSFSGFSLSTSG MGVGWIRQPSGKGLEWLAHIWWDDVKSYNPALKSRLTISKDTSSSQVFLRIASVDT ADTATYYCARIGDGYYSFDYWGQGTTLTVSSAKTTPPSVYPLAPGSAAQTNSMVTL GCLVKGYFPEPVTVTWN (The highlighted regions relate respectively to CDR 2, CDR3, and CDR 4).

### Nucleotide sequence of NovelMed kappa chain variable region NM14G11.9

### Amino acid sequence of the Kappa light chain

MHHTSMGIKMESQIQVFVFVFLWLSGVDGDIVMTQSHKFMSTSVGDRV SISCKASQDVSDAVAWFQQKPGQSPKLLIYSPSYRYTGVPDRFTGSGSGTDFTFTISSV QAEDLAVYYCQQHYSTPWTFGGGTKLEIKRADAAPTV (The highlighted regions relate respectively to CDR 1, CDR2, and CDR3).

## Claims

1. Use of an antibody or fragment thereof for inhibiting alternative complement pathway activation in the blood of a mammal, the antibody or fragment thereof specifically binding to properdin and inhibiting the alternative complement pathway activation in the mammal by inhibiting the properdin binding to C5, wherein the antibody or fragment thereof comprises a heavy chain variable domain comprising the three CDRs of SEQ ID NO: 1, SEQ ID NO: 2 and SEQ ID NO: 3 and a light chain variable domain comprising the three CDRs of SEQ ID NO: 4, SEQ ID NO: 5 and SEQ ID NO: 6, wherein said antibody binds to properdin with a binding affinity of < 100pM.

2. The use of claim 1, wherein the antibody or fragment thereof inhibits the formation of heteropolymers of P-C3bBb.

3. The use of claim 1 or 2, wherein the antibody or fragment thereof specifically binds to an epitope on properdin defined by SEQ ID NO 15 (AFAYQKRSGGLCQPCRSPRWSLWS).

4. The use of any of the preceding claims, wherein the antibody is a chimeric, recombinant, humanized, de-immunized or humaneered antibody.

5. The use of any of the preceding claims, wherein the antibody or fragment thereof inhibits the alternative pathway without inhibiting the classical pathway.

6. The use of any of the preceding claims, wherein the antibody or fragment thereof reduces the level of free properdin in a mammal.

7. The use of any of the preceding claims, wherein the heavy chain variable domain comprises an amino acid sequence selected from the group consisting of SEQ ID NO: 7, SEQ ID NO: 9, SEQ ID NO: 11 and SEQ ID NO: 13.

8. The use of any of the preceding claims, wherein the light chain variable domain comprises an amino acid sequence selected from the group consisting of SEQ ID NO: 8, SEQ ID NO: 10, SEQ ID NO: 12 and SEQ ID NO: 14.

9. The use of any of the preceding claims, wherein the heavy chain variable domain comprises the amino acid sequence of SEQ ID NO: 7 and the light chain variable domain comprises the amino acid sequence of SEQ ID NO: 8.

10. The use of any of the preceding claims, wherein the heavy chain variable domain comprises the amino acid sequence of SEQ ID NO: 9 and the light chain variable domain comprises the amino acid sequence of SEQ ID NO: 10.

## Patentansprüche

1. Verwendung eines Antikörpers oder Fragments davon zur Hemmung der Aktivierung des alternativen Komplementwegs im Blut eines Säugetiers, wobei der Antikörper oder das Fragment davon spezifisch an Properdin bindet und die Aktivierung des alternativen Komplementwegs durch Hemmung der Bindung von Properdin an C5 hemmt, wobei der Antikörper oder das Fragment davon umfasst eine variable Domäne der schweren Kette, die die drei CDRs gemäß SEQ ID NO: 1, SEQ ID NO: 2 und SEQ ID NO: 3 umfasst und eine variable Domäne der leichten Kette, die die drei CDRs gemäß SEQ ID NO: 4, SEQ ID NO: 5 und SEQ ID NO: 6 umfasst, wobei der Antikörper mit einer Bindungsaffinität von < 100pM an Properdin bindet.

2. Verwendung nach Anspruch 1, wobei der Antikörper oder das Fragment davon die Bildung von Heteropolymeren von P-C3bBb hemmt.

3. Verwendung nach Anspruch 1 oder 2, wobei der Antikörper oder das Fragment davon spezifisch an ein Epitop in Properdin bindet, das durch SEQ ID NO 15 (AFAYQKRSGGLCQPCRSPRWSLWS) definiert ist.

4. Verwendung nach einem der vorhergehenden Ansprüche, wobei der Antikörper ein chimärer, rekombinanter, humanisierter, deimmunisierter oder humaneered Antikörper ist.

5. Verwendung nach einem der vorhergehenden Ansprüche, wobei der Antikörper oder das Fragment davon den alternativen Weg hemmt, ohne den klassischen Weg zu hemmen.

6. Verwendung nach einem der vorhergehenden Ansprüche, wobei der Antikörper oder das Fragment davon die Menge an freiem Properdin in einem Säugetier reduziert.

7. Verwendung nach einem der vorhergehenden Ansprüche, wobei die variable Domäne der schweren Kette eine Aminosäuresequenz ausgewählt aus der Gruppe bestehend aus SEQ ID NO: 7, SEQ ID NO: 9, SEQ ID NO: 11 und SEQ ID NO: 13 umfasst.

8. Verwendung nach einem der vorhergehenden Ansprüche, wobei die variable Domäne der leichten Kette eine Aminosäuresequenz ausgewählt aus der Gruppe bestehend aus SEQ ID NO: 8, SEQ ID NO: 10, SEQ ID NO: 12 und SEQ ID NO: 14 umfasst.

9. Verwendung nach einem der vorhergehenden Ansprüche, wobei die variable Domäne der schweren Kette die Aminosäuresequenz nach SEQ ID NO: 7 und die variable Domäne der leichten Kette die Aminosäuresequenz nach SEQ ID NO: 8 umfasst.

10. Verwendung nach einem der vorhergehenden Ansprüche, wobei die variable Domäne der schweren Kette die Aminosäuresequenz nach SEQ ID NO: 9 und die variable Domäne der leichten Kette die Aminosäuresequenz nach SEQ ID NO: 10 umfasst.

## Revendications

1. Utilisation d'un anticorps ou d'un fragment de celui-ci pour inhiber l'activation de la voie alterne du complément dans le sang d'un mammifère, l'anticorps ou le fragment de celui-ci se liant spécifiquement à la properdine et inhibant l'activation de la voie alterne du complément chez le mammifère en inhibant la liaison de la properdine à C5, sachant que l'anticorps ou le fragment de celui-ci comprend un domaine variable de chaîne lourde comprenant les trois CDR de SEQ ID N° : 1, SEQ ID N° : 2 et SEQ ID N° : 3 et un domaine variable de chaîne légère comprenant les trois CDR de SEQ ID N° : 4, SEQ ID N° : 5 et SEQ ID N° : 6, sachant que ledit anticorps se lie à la properdine avec une affinité de liaison de < 100 pM.

2. L'utilisation de la revendication 1, sachant que l'anticorps ou le fragment de celui-ci inhibe la formation d'hétéropolymères de P-C3bBb.

3. L'utilisation de la revendication 1 ou 2, sachant que l'anticorps ou le fragment de celui-ci se lie spécifiquement à un épitope sur la properdine défini par SEQ ID N° 15 (AFAYQKRSGGLCQPCRSPRWSLWS).

4. L'utilisation de l'une quelconque des revendications précédentes, sachant que l'anticorps est un anticorps chimérique, recombinant, humanisé, désimmunisé ou Humaneered.

5. L'utilisation de l'une quelconque des revendications précédentes, sachant que l'anticorps ou le fragment de celui-ci inhibe la voie alterne sans inhiber la voie classique.

6. L'utilisation de l'une quelconque des revendications précédentes, sachant que l'anticorps ou le fragment de celui-ci réduit le niveau de properdine libre chez un mammifère.

7. L'utilisation de l'une quelconque des revendications précédentes, sachant que le domaine variable de chaîne lourde comprend une séquence d'acide aminé sélectionnée dans le groupe constitué par SEQ ID N° : 7, SEQ ID N° : 9, SEQ ID N° : 11 et SEQ ID N° : 13.

8. L'utilisation de l'une quelconque des revendications précédentes, sachant que le domaine variable de chaîne légère comprend une séquence d'acide aminé sélectionnée dans le groupe constitué par SEQ ID N° : 8, SEQ ID N° : 10, SEQ ID N° : 12 et SEQ ID N° : 14.

9. L'utilisation de l'une quelconque des revendications précédentes, sachant que le domaine variable de chaîne lourde comprend la séquence d'acide aminé de SEQ ID N° : 7 et le domaine variable de chaîne légère comprend la séquence d'acide aminé de SEQ ID N° : 8.

10. L'utilisation de l'une quelconque des revendications précédentes, sachant que le domaine variable de chaîne lourde comprend la séquence d'acide aminé de SEQ ID N° : 9 et le domaine variable de chaîne légère comprend la séquence d'acide aminé de SEQ ID N° : 10.
